(19) **Europäisches Patentamt / European Patent Office / Office européen des brevets**

(11) **EP 2 629 848 B1**

(12) **EUROPEAN PATENT SPECIFICATION**

(45) Date of publication and mention
of the grant of the patent:
**08.10.2014 Bulletin 2014/41**

(51) Int Cl.:
*A61N 7/00* *(2006.01)*     *A61M 25/00* *(2006.01)*
*A61B 17/00* *(2006.01)*     *A61M 25/10* *(2013.01)*
*G01N 29/44* *(2006.01)*     *G01N 29/46* *(2006.01)*

(21) Application number: **11782223.9**

(22) Date of filing: **18.10.2011**

(86) International application number:
**PCT/IB2011/054639**

(87) International publication number:
**WO 2012/052925 (26.04.2012 Gazette 2012/17)**

(54) **AN ULTRASOUND TRANSCEIVER AND CONTROL OF A THERMAL DAMAGE PROCESS**

ULTRASCHALL-TRANSCEIVER UND STEUERUNG EINES WÄRMESCHADENSPROZESSES

EMETTEUR-RÉCEPTEUR D'ULTRASONS ET MAÎTRISE D'UN PROCESSUS
D'ENDOMMAGEMENT THERMIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **16.03.2011   US 201161453239 P
16.03.2011   US 201113049022
18.10.2010   US 393947 P**

(43) Date of publication of application:
**28.08.2013   Bulletin 2013/35**

(73) Proprietor: **Cardiosonic Ltd.
69710 Tel-Aviv (IL)**

(72) Inventors:
• **SVERDLIK, Ariel**
  **69547 Tel-Aviv (IL)**
• **SHABTAY, Or**
  **20110 Doar-Na Bikat Beit HaKerem (IL)**
• **SHEIN-IDELSON, Mark**
  **62265 Tel-Aviv (IL)**
• **MALAFRIEV, Oleg**
  **76390 Rehovot (IL)**

(74) Representative: **Jansen, Cornelis Marinus et al
V.O.
Johan de Wittlaan 7
2517 JR Den Haag (NL)**

(56) References cited:
**US-A- 5 657 760**

• **BAILEY M R ET AL: "Cavitation detection during
shock-wave lithotripsy", ULTRASOUND IN
MEDICINE AND BIOLOGY, NEW YORK, NY, US,
vol. 31, no. 9, 1 September 2005 (2005-09-01),
pages 1245-1256, XP027605630, ISSN: 0301-5629
[retrieved on 2005-09-01]**
• **CATTAFESTA L N ET AL: "Acoustic proximity
ranging in the presence of secondary echoes",
IEEE TRANSACTIONS ON INSTRUMENTATION
AND MEASUREMENT, IEEE SERVICE CENTER,
PISCATAWAY, NJ, US, vol. 52, no. 5, 1 October
2003 (2003-10-01), pages 1593-1605,
XP011102759, ISSN: 0018-9456, DOI:
10.1109/TIM.2003.817907**

EP 2 629 848 B1

**Description**

FIELD AND BACKGROUND OF THE INVENTION

The present invention, in some embodiments thereof, relates to an ultrasound transceiver and to control of an ablation or thermal damage process to a tissue and, more particularly, but not exclusively, to use of the transceiver in simultaneous monitoring and ablation of nearby tissue by monitoring the distance to the tissue walls, and use of change in distance between tissue walls as an indicator of progress of the thermal damage.

For the purposes of ablation of tissue it is desirable to have an ultrasound transmitter which is undamped, because any damping reduced efficiency.

For the purpose of detection of effects, a damped receiver is desirable, since without damping, ringing occurs, making signals hard to read.

In the case where the same device is used both for ablation and detection, the situation is particularly bad, since the transmitted signals cause considerable ringing in the undamped device.

The problem is to detect the wall of an artery, or any other tissue, using an ultrasonic transceiver which is also performing the ablation and which thus must be undamped because damping will reduce efficiency. Low efficiency leads to increased heating, which can be harmful in confined spaces such as blood vessels.

Since the detector is undamped there is considerable noise due to ringing. The ringing is present in the sensor and is also transmitted to the tissues causing reflections of its own, all this interfering with the signal it is intended to detect.

As a consequence, the signal from the artery wall is drowned out by noise due to ringing and the primary echo is hard to discern. Nevertheless, detecting the echo is highly desirable in order to monitor the ablation treatment.

SUMMARY OF THE INVENTION

An embodiment of the present invention may analyze a sequence of sample signals. The primary echo has a fixed relationship to the excitation signal, whereas the ringing signals do not. Typically the primary echo shares a main frequency component with the excitation signal although not the phase and not the amplitude. The relationship is used to distinguish the primary echo from ringing and from secondary echoes. According to an aspect of some embodiments of the present invention there is provided an ultrasonic transceiver apparatus for intracorporeal use, the apparatus comprising:

an undamped ultrasonic transceiver for placing in a confined intracorporeal space, the transceiver having an instantaneous excitation frequency and for receiving excitation at the excitation frequency to produce an ultrasonic ablation beam for ablating surrounding tissues and being further for receiving excitation by primary echo signals returning from surrounding tissues;
a signal processor connected to the transceiver configured to isolate the primary echo signals from ringing, secondary echoes and extraneous noise also received from the transceiver, the signal processor using presence or absence of the instantaneous excitation frequency as an isolation criterion.

In an embodiment, the signal processor is configured with an instantaneous frequency estimator to obtain an envelope of received signal minus excitation signal from the undamped ultrasonic transceiver and to use a global phase and local slopes thereof as an estimate of the instantaneous frequency, and further comprising an isolator unit for isolating signal segments whose instantaneous frequency approaches the characteristic frequency as segments containing primary echoes.

An embodiment may comprise a window unit for windowing the received signal using a windowing length chosen to provide windows with an expectation of a single primary echo.

In an embodiment, the signal processor is further configured to find a point of appearance of a primary echo in a received signal by successively dividing the curve and fitting to a linear functions and calculating a point at which a corresponding error function is minimized.

An embodiment may be configured with a location unit to determine a distance to a first feature wall from the point of appearance.

In an embodiment, the location unit is configured to use a second point of appearance of a further primary echo to determine a distance to a second feature wall, the signal processor further comprising a monitoring unit for monitoring a distance between the first feature wall and the second feature wall as an indicator of ablation progress.

In an embodiment, the signal processor comprising a convolution unit for convolving an excitation signal with the received signal to carry out the isolation of the primary echo.

In an embodiment, the signal processor comprises a Fourier component analyzer for isolating segments having a principle Fourier component which corresponds to a body-characteristic frequency.

In an embodiment, the signal processor comprises a coherent summation unit for carrying out data summation such as to preserve amplitude and shift signals to a same phase.

In an embodiment, the coherent summation unit is configured to perform coherent summation, the coherent summation comprising building an auxiliary matrix of phase weights, making a Hilbert transform and multiplying to bring all the signal to the same phase, therewith to create an in-phase sum.

An embodiment may comprise a reference subtracting unit configured to subtract a reference from the transceiver signal by averaging several signal samples to eliminate unstable component.

According to a second aspect of the present disclosure there is provided an ultrasonic transceiver method for intracorporeal use, the method comprising:

placing an undamped ultrasonic transceiver in a confined intracorporeal space, the transceiver having a characteristic frequency; exciting the transceiver at an instantaneous excitation frequency to produce an ultrasonic ablation beam for ablating surrounding tissues using ablation pulses;

at intervals between the ablation pulses providing monitoring excitation to elicit primary echo signals returning from surrounding tissues; and

isolating the primary echo signals from ringing, secondary echoes and extraneous noise also received from the transceiver using presence or absence of the instantaneous excitation frequency as an isolation criterion.

In an embodiment, the isolation comprises obtaining an envelope of received signal minus excitation signal from the undamped ultrasonic transceiver and using a global phase and local slopes as an estimate of the instantaneous frequency, and isolating those signal segments whose frequency approaches the instantaneous excitation frequency.

An embodiment may comprise windowing the received signal using a windowing length chosen to provide windows with an expectation of a single primary echo.

An embodiment may be configured to find a point of appearance of a primary echo in a received signal by:

successively dividing the curve;

fitting to a linear functions; and

calculating a point at which a corresponding error function is minimized.

An embodiment may comprise determining a distance to a first feature wall from the point of appearance.

An embodiment may comprise using a second point of appearance of a further primary echo to determine a distance to a second feature wall, and monitoring a distance between the first feature wall and the second feature wall as an indicator of ablation progress.

An embodiment may comprise convolving an excitation signal with the received signal to carry out the isolation of the primary echo.

An embodiment may comprise isolating segments having a principle Fourier component which corresponds to a body characteristic frequency.

An embodiment may comprise carrying out coherent data summation such as to preserve amplitude and shift signals to a single phase.

In an embodiment, the coherent summation comprises:

making an auxiliary weights matrix evaluation;

carrying out a Hilbert transformation;

multiplication to bring all signals to the same phase; and

performing an in-phase summation.

According to a third aspect of the present invention there is provided an ultrasonic transceiver apparatus for intracorporeal use, the apparatus comprising:

an undamped ultrasonic transceiver for placing in a confined intracorporeal space, the transceiver having a characteristic frequency and for receiving excitation at the characteristic frequency to produce an ultrasonic ablation beam for ablating surrounding tissues and being further for receiving excitation by primary echo signals returning from surrounding tissues;

a signal processor connected to the transceiver configured to isolate the primary echo signals from ringing, secondary echoes and extraneous noise also received from the transceiver, the signal processor using correlation with a body-characteristic frequency as an isolation criterion.

In an embodiment, the body characteristic frequency may be pulse or breathing rate.

In an embodiment, the signal processor is configured to obtain a power spectrum of a signal extracted from the transceiver and to identify the primary echoes from peaks in the power spectrum at the body-characteristic frequency.

An embodiment may comprise a coherent summation unit or a convolution unit or both.

According to a fourth aspect of the present disclosure there is provided a method of providing controlled thermal damage to a tissue, comprising:

identifying locations of boundary walls of the tissue;

applying energy to the tissue;

during the applying, monitoring changes in locations of the boundary walls as indicators of the application of energy to the tissue, for example thermal shrinkage of the tissue; and

controlling the thermal energy according to the effect, for example, thermal shrinkage.

In an embodiment, the monitoring and applying are carried out from within a blood vessel, and/ or using ultrasonics.

In this fourth aspect of the invention, a known ablation device may be used in conjunction with an ultrasonic detector.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

Implementation of the system of embodiments of the invention can involve performing or completing selected tasks manually, automatically, or a combination thereof. Moreover, according to actual instrumentation and equipment of embodiments of the system of the invention, several selected tasks could be implemented by hardware, by software or by firmware or by a combination thereof using an operating system.

For example, hardware for performing selected tasks according to embodiments of the invention could be implemented as a chip or a circuit. As software, selected tasks according to embodiments of the invention could be implemented as a plurality of software instructions being executed by a computer using any suitable operating system. In an exemplary embodiment of the invention, one or more tasks according to exemplary embodiments of method and/or system as described herein are performed by a data processor, such as a computing platform for executing a plurality of instructions. Optionally, the data processor includes a volatile memory for storing instructions and/or data and/or a non-volatile storage, for example, a magnetic hard-disk and/or removable media, for storing instructions and/or data. Optionally, a network connection is provided as well. A display and/or a user input device such as a keyboard or mouse are optionally provided as well. The invention is defined in the claims.

BRIEF DESCRIPTION OF THE DRAWINGS

The patent or application file contains at least one drawing executed in color. Copies of this patent or patent application publication with color drawing(s) will be provided by the Office upon request and payment of the necessary fee.

Some embodiments of the invention are herein described, by way of example only, with reference to the accompanying drawings. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.

In the drawings:

FIG. 1 is a simplified schematic block diagram illustrating ultrasonic transceiver apparatus according to an embodiment of the present invention;

FIG. 2 is a simplified graph showing a raw signal received from the transceiver and the same signal after reference subtraction;

FIG. 3 is a simplified graph showing the signal of FIG. 2 after averaging and after coherent summation;

FIG. 4 is a simplified graph showing the raw signal after noise subtraction and after double coherent summation and convolution;

FIGs. 5A-5B and 6-8 illustrate experimental data for a measured distance of 0.19mm;

FIGs. 9A-9B and 10-12 illustrate experimental data for a measured distance of 1.19mm;

FIGs. 13A-13B and 14-16 illustrate experimental data for a measured distance of 1.69mm;

FIGs. 17A-17B and 18-20 illustrate experimental data for a measured distance of 2.19mm;

FIGs. 21A-21B and 22-24 illustrate experimental data for a measured distance of 2.69mm;

FIGs. 25A-25B and 26-28 illustrate experimental data for a measured distance of 3.19mm;

FIGs. 29A-29B and 30-32 illustrate experimental data for a measured distance of 3.69mm;

FIGs. 33A-33B and 34-36 illustrate experimental data for a measured distance of 4.19mm;

FIGs. 37A-37B and 38-40 illustrate experimental data for a measured distance of 4.69mm;

FIGs. 41A-41B and 42-44 illustrate experimental data for a measured distance of 5.19mm;

FIG. 45 is a correlation graph derived from the experiments of FIGs. 5A to 45, and indicating a very high correlation between actual and measured distances;

FIG. 46 is a simplified flow chart illustrating the process of coherent summation according to an embodiment of the present invention;

FIG. 47 is a flow chart showing in greater detail the actual transceiver frequency evaluation of FIG. 46;

FIG. 48 is a simplified graph illustrating division of envelope sections according to amplitude of the principle Fourier component matching the device resonance, and showing the selection of the sections having the highest amplitude, according to an embodiment of the present invention;

FIG. 49 illustrates ringing following an excitation signal, with primary echoes hidden in the ringing;

FIG. 50 shows sampling to obtain vectors, undamped ringing with echoes included, and a power spectrum graph at the pulse frequency, where peaks in the power spectrum graph indicate features in the primary echo; and

FIG. 51 is a simplified flow chart illustrating a procedure for obtaining the power spectrum graph of FIG. 50.

## DESCRIPTION OF SPECIFIC EMBODIMENTS OF THE INVENTION

The present invention, in some embodiments thereof, relates to an ultrasound transceiver, in particular for use in confined spaces such as blood vessel to carry out self-monitored ablation on surrounding tissues.

The problem is to detect the wall of an artery (or any other tissue) using an ultrasonic detector which must be undamped because damping will reduce efficiency.

In general the effectiveness of an ablation process can be determined by measuring the distance between outer and inner walls of a tissue being ablated. Specifically, as the tissue is ablated, the distance between the outer and inner walls falls, so sequential monitoring of the distance is a way of measuring the effectiveness of the ablation process.

Since the detector is undamped there is considerable noise due to ringing. The ringing is present in the sensor and is also transmitted to the tissues causing reflections of its own, all this interfering with the signal it is intended to detect.

The issue is that the signal from the artery wall is drowned out by noise and the ringing. We know however that the signal from the tissue wall has certain characteristics. The primary ringing tends to share a main frequency with the excitation signal, although the phase will differ depending on the distance. Thus the frequency can be used to distinguish primary echoes from ringing and from secondary echoes as well as general noise.

Once the primary echoes have been distinguished then it is possible to monitor the thermic effectiveness of the treatment by locating the inner and outer walls and monitoring the change in distance between them.

More generally, detecting the primary echoes allows them to be used in an analysis of the entire artery wall tissue signal, and not only the face of the wall that is touching the blood.

Various methods are given in the disclosure for analyzing the primary echoes.

Since the transceiver is a narrow band device, it irradiates mainly at its Eigen frequency. Furthermore, voltages generated by returned pressure waves are again filtered by the transceiver, so that the primary reflected signal is an almost pure harmonic oscillation, albeit with variable amplitude. It is reasonable to expect that transient-only portions of the signal are characterized by different frequencies.

The present embodiments further include a means of providing controlled thermal damage to a tissue, by identifying locations of boundary walls of the tissue, applying energy to the tissue, and monitoring changes in locations of the boundary walls as indicators of the application of energy to the tissue, for example thermal shrinkage of the tissue; Then the application of the thermal energy can be controlled y according to the detected effect.

In an embodiment, the monitoring and applying are carried out from within a blood vessel, and/ or using ultrasonics. The above may be carried out using the transceiver described herein but alternatively, a known ablation device may be used in conjunction with an ultrasonic detector.

It is noted that the effect on the tissue is not necessarily shrinkage. It may instead be thickening due to heating or to some physiological response. Nevertheless it is the change in size or shape of the tissue, as determined by changes in tissue wall location determined by non-imaging or imaging of tissue, that is used to control the process.

The present embodiments relate to non-focused ultrasound, or to focused ultrasound or to r.f. based systems.

Before explaining at least one embodiment of the invention in detail, it is to be understood that the invention is not necessarily limited in its application to the details of construction and the arrangement of the components set forth in the following description and/or illustrated in the drawings and/or the Examples. The invention is capable of other embodiments or of being practiced or carried out in various ways.

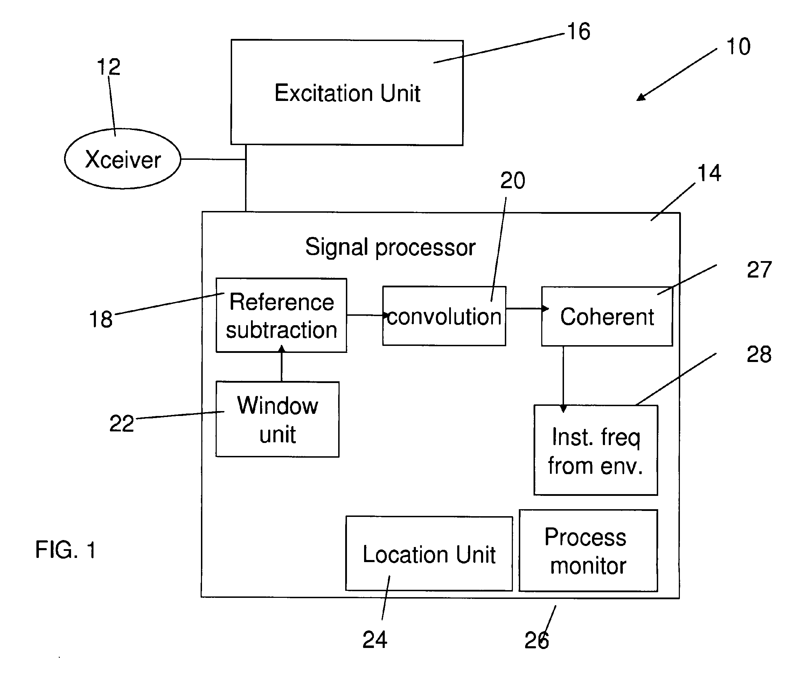

Referring now to the drawings, Figure 1 illustrates an ultrasonic transceiver apparatus 10 primarily intended for intracorporeal use, according to one embodiment of the present invention. A transceiver 12 is designed to be injected into blood vessels and like confined spaces within the body, particularly with a view to flowing towards locations where tissue ablation is required, and to direct ultrasound energy towards the tissue to be ablated. In between ablations the transceiver is excited with monitoring signals to use ultrasound energy for a different purpose, the monitoring of the surrounding tissues to make sure that the ablation is being carried out effectively.

As mentioned in the background ablation takes place in the body, where heating of the wrong tissues is undesirable. Thus the transceiver is required to be as efficient as possible and thus to be undamped. Monitoring however requires a damped transceiver since reading the echoes is conventionally only possible once the excitations have died down. Using different transceiver surfaces for monitoring and ablation respectively is also not ideal since it is difficult to guarantee that the tissue being monitored is the same as the tissue being ablated.

The present embodiments thus use a single undamped transceiver for both monitoring and ablation. Structures are provided for isolating the primary reflections from ringing due to the excitation signal and due to secondary reflections from the ringing as well as assorted other noise. The undamped ultrasonic transceiver may be a narrow band transceiver having a characteristic excitation frequency so that all excitations are at that characteristic frequency. The primary echoes tend to share the excitation frequency, albeit at variable phases and amplitudes, whereas general ringing and tend to be at other g frequencies.

A signal processor 14 is connected to the transceiver 12 to isolate the primary echo signals from the ringing, secondary echoes and extraneous noise also received from the transceiver. The signal processor uses presence or absence of the characteristic frequency as an isolation criterion.

An excitation unit 16 provides an excitation signal for the transceiver. The excitation may be high power for the ablation or low power for monitoring.

The signal processor may have reference subtraction unit 18 which subtracts the excitation signal, from the transceiver 12.

As will be discussed in greater detail below, the size of the segments may be chosen so that only one primary echo need be found per segment. Otherwise the presence of multiple reflections makes analysis more difficult. The distances between tissue walls are known in general terms so segment lengths are chosen to represent distances smaller than the distance between walls. Thus a window unit 22 windows the received signal using a windowing length chosen to provide segments with an expectation of a single primary echo.

There are a number of alternative ways in which the segment isolator can find primary echoes.

A convolution unit 20 convolves an excitation signal with the received signal to isolate only those waveforms having a high coherence with the excitation signals.

Following convolution is coherent summation. A coherent summation unit 27 carries out data summation such as to preserve amplitude and shift signals to the same phase. The coherent summation unit may build an auxiliary matrix and then multiplies it with previously carried out Hilbert transform to shift the obtained complex analytic signals to the same phase and to make in-phase (coherent) summation Coherent summation is discussed in greater detail below.

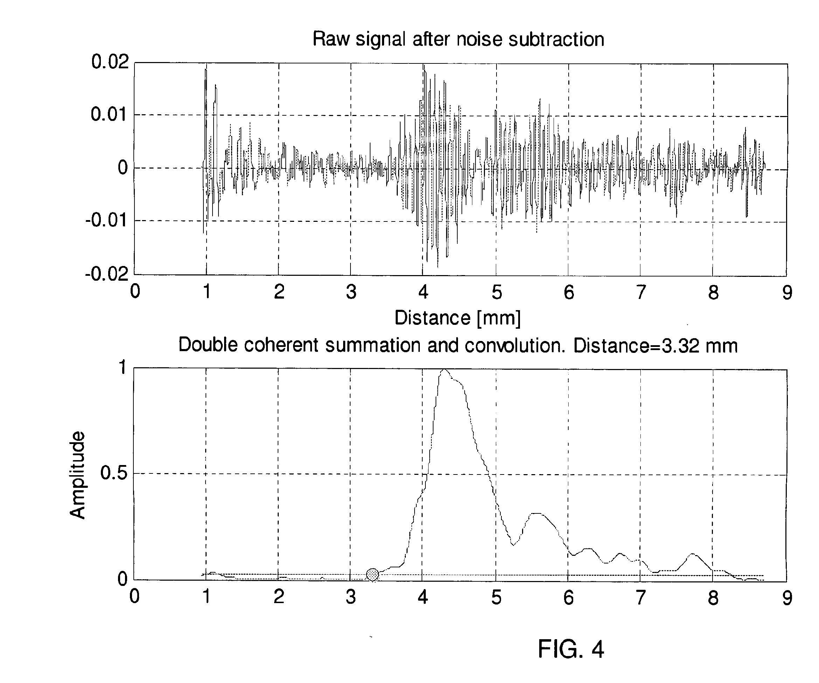

Although it is possible to use convolution alone or coherent summation alone it is also possible to combine operation of the above units. Fig. 4 below discusses double coherent summation and convolution as a way to obtain the primary echoes.

The above processes may involve obtaining a reference signal, based on the actual excitation. Reference subtracting unit 18 may subtract a reference from the transceiver signal by averaging several signal samples (particular records), simply to eliminate unstable components and obtain the actual excitation. Optionally, one can use as a reference a separate signal where echoes do not arrive until after the ringing dies out. That is, a part of the signal where the ringing has disappeared but echo has not appeared yet can be used as a reference. A separate reference signal is known to be free of echoes in the region of interest. Thus, its subtraction from the particular signal reliably provides us with almost pure echo, except for the noise component.

Finally, after double coherent summation and convolution, an envelope is obtained.

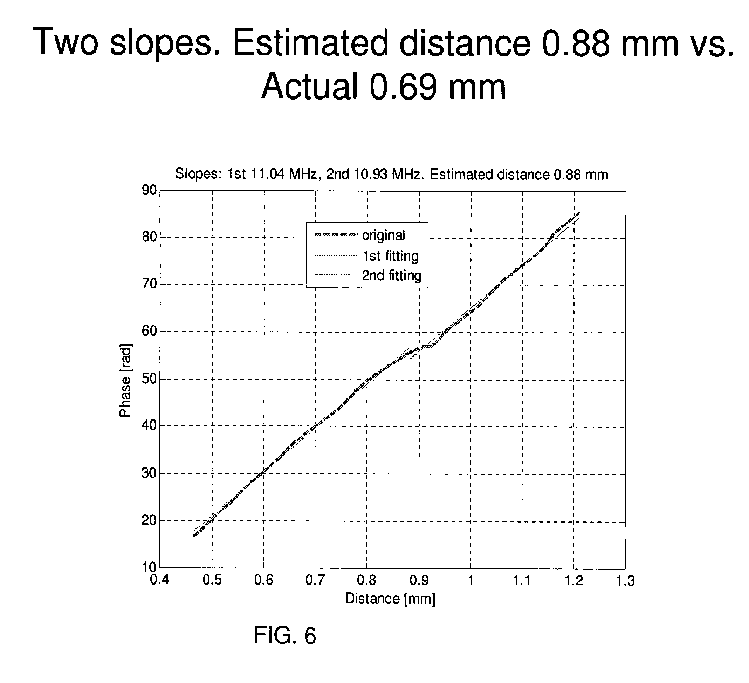

With the envelope, the signal processor may find a point of appearance of a primary echo in the segment by successively dividing the envelope curve while fitting to a linear function and calculating a point at which a corresponding error function is minimized. Such a process is illustrated in Fig. 6 below.

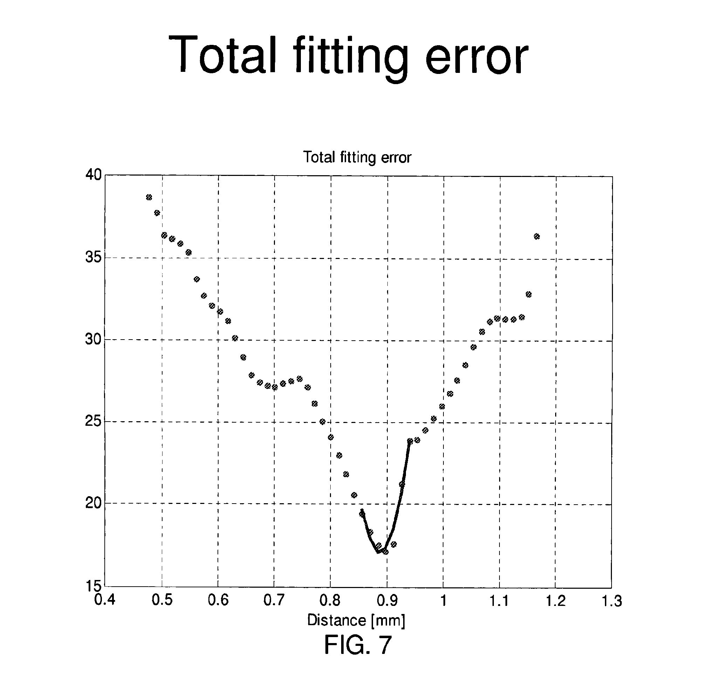

The point of error minimization is most probably the point of onset of the primary echo and indicates the distance to a structure such as a tissue wall. Such an error minimization is shown in Fig. 7 discussed below. A location unit 24 determines the distance to a first feature wall based on the point of appearance.

The location unit may then use a second point of appearance of a further primary echo to determine a distance to a second feature wall. A process monitoring unit then calculates and monitors the distance between the first feature wall and the second feature wall as an indicator of ablation progress. As the ablation progresses the wall typically shrinks as the tissue dries out.

An alternative to the above is Fourier component analysis, based not on the excitation frequency of the transceiver, but on frequencies characteristic of the body, such as pulse rate or breathing rate.

Using Fourier component analysis, the signal processor uses correlation with a body-characteristic frequency as an isolation criterion. One way of doing this is to obtain a power spectrum of the signal extracted from the transceiver and to identify the primary echoes from peaks in the power spectrum at the body-characteristic frequency.

The Fourier component analysis may be used instead of or in addition to coherent summation and to convolution using a convolution unit.

The above processes are now considered in greater detail.

As mentioned, an envelope of the oscillating signal is obtained, and a global phase and its local slopes are used as estimations of instantaneous frequency (actually, instantaneous frequency is local slope itself). Also instantaneous frequency is estimated from the entire signal in analytical form, as time derivative of phase. The appearance of a primary reflection signal is then recognized within the overall envelope as a transition at an instantaneous frequency of the signal. The transceiver frequency remains constant.

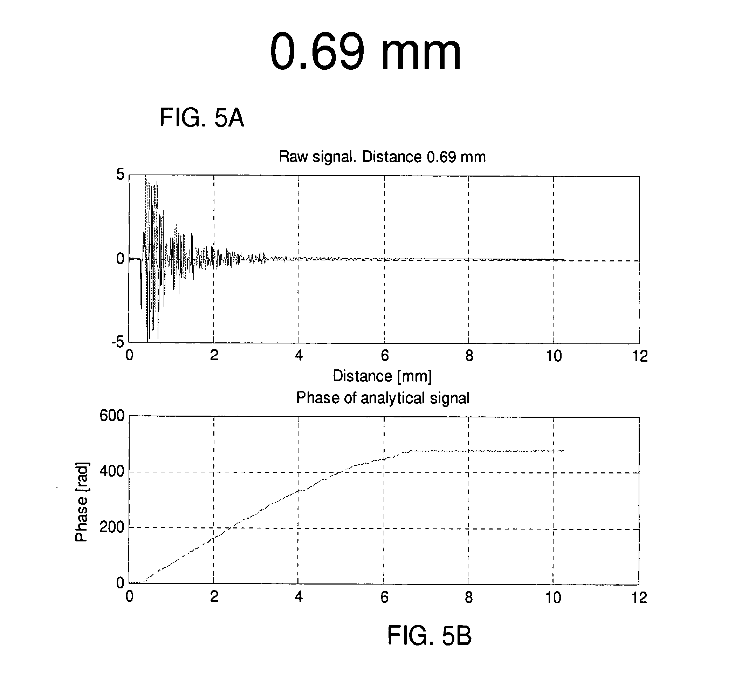

The Hilbert transform is carried out on a residual signal, after reference subtraction. The results are used for coherent summation of original signals. signal construction the Hilbert transform effectively creates a signal shifted by a quarter of a period with respect to the original signal. For a true harmonic signal, the phase of the obtained complex analytic signal - see Fig. 5B, grows linearly in time. If a new harmonic component appears in the signal, it may be revealed by a change of the phase - time curve slope.

The appearance of a new harmonic component may be taken as the indication of a new feature that is being detected, say the near surface of a tissue in question, and a second new harmonic component may indicate the far surface of the tissue in question artery wall.

Finding the Primary Echo

To find the instant of new component appearance, an algorithm for minimum total error may be used. As illustrated in Fig. 6, the entire curve may be divided in two parts and each part may then be fitted to a linear function. We consider double fitting of the entire error as a function of the separation point. The assumption is that for the true separation point between two frequency regions the overall error reaches its minimum. In general, a change in best slope indicates the appearance of a new event. The point where the new harmonic is added turns out to be derivable from the error function. The overall error of the two curve fitting operations is a function of the point of appearance of the new harmonic, and minimization of the overall error may indicate the point. Fig. 7 illustrates such an error function with a clear minimization point.

An issue arises in that the result of the above-described procedure is sensitive to the length of the data subjected to analysis. The above-described procedure works best when a stretch of data contains just a single point at which a new harmonic is added. But, in real life there are more, and surfaces have inner and outer walls which each give rise to separate reflections and there are second order reflections and also noise. Thus, the presence of a second reflection or say a long portion of noise, negatively affects the accuracy. The problem may be solved by windowing, analyzing the received data in short chunks, as will be explained in greater detail below.

Correlation approach

The excitation voltage generates mechanical oscillations in the transceiver, which irradiate pressure waves into surrounding media. Return pressure waves generate a reflection voltage signal. It is reasonable to expect that the reflection retains at least a partial correlation with the excitation voltage. An alternative or additional approach is just to convolve part of the excitation signal with the rest of the data. The reflection may thus be amplified according to the correlation and become more recognizable.

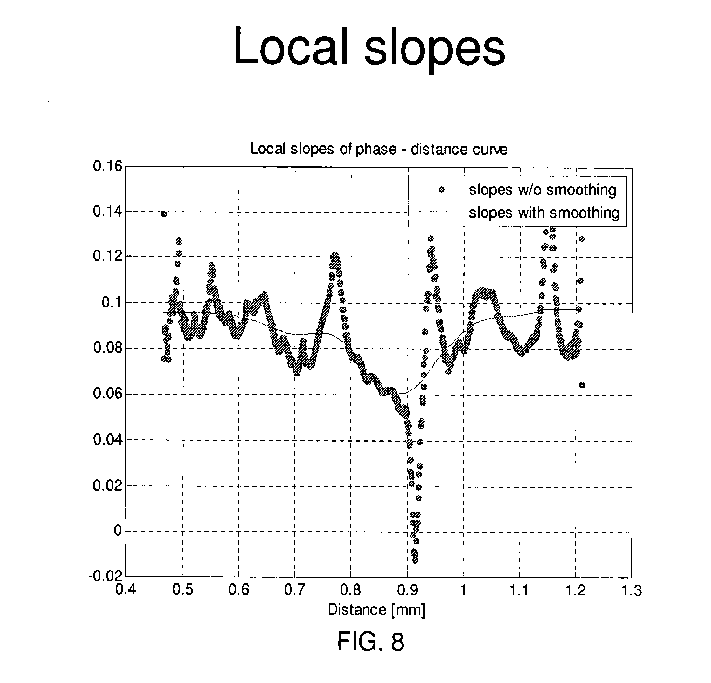

Reference is now made to Fig. 8, which illustrates local slopes of a phase-distance curve. Over the full phase - time (distance) curve one can evaluate local standard statistical parameter correlation coefficients. For perfectly linear dependence between two values the correlation coefficient is $\pm1$. For noisy data or for nonlinear dependence, the absolute value is less than 1. A stable and significant decrease of correlation coefficient serves as a reliable sign of useless noisy data and allows the irrelevant signal to be excluded.

The use of correlation coefficients can thus be used to find parts of the data where there are strong echoes and ignore parts where such strong echoes are absent.

Experimental Calibration

The present embodiments were applied experimentally to estimate distance within the interval 0.19 - 5.19 mm.

Two types of experiments were carried out, *in vitro* and *in vivo.*

In *in vitro* experiments, it is possible to control distance between transceiver and reflective object (metal plate) by means of a positioning device with a ruler and set it in parallel to the transceiver surface. Accuracy of 0.1 mm is available. In the following, in vitro experimentation is used to generate a calibration curve between measured and actual distances. That is, to the experiment finds slope and intercept. The slope is expected to be close to 1. The intercept allows for elimination of systematic error in the ruler reading. Indeed, the intercept is not the interesting point, although technically necessary. The indication of evaluation correctness is that the evaluated points lie on a straight line with a slope close to 1.

Several remote points with reliable separation between transient and reflection were tested and an offset (the above-referred to intercept) was estimated. A true distance can then be used which is the sum of the ruler reading and offset.

Sample Equivalents

In the time domain, the sample equivalent is a reciprocal sampling frequency. For 800 MHz, it is 1.25 ns.

In the space domain, the sample equivalent is half of the sampling period multiplied by sound velocity. Its interpolated value at 23° C is 1489 m/s. Thus, a distance equivalent is 9.306-10-4 mm.

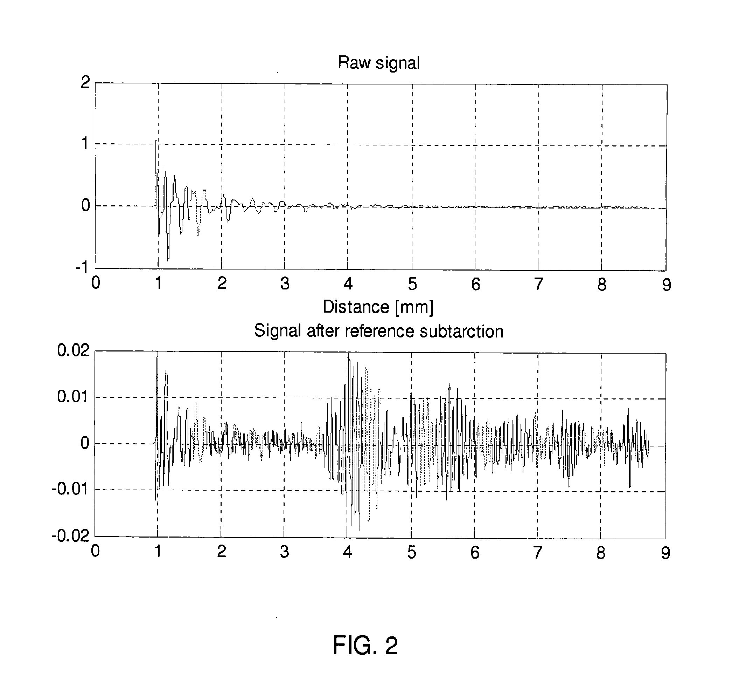

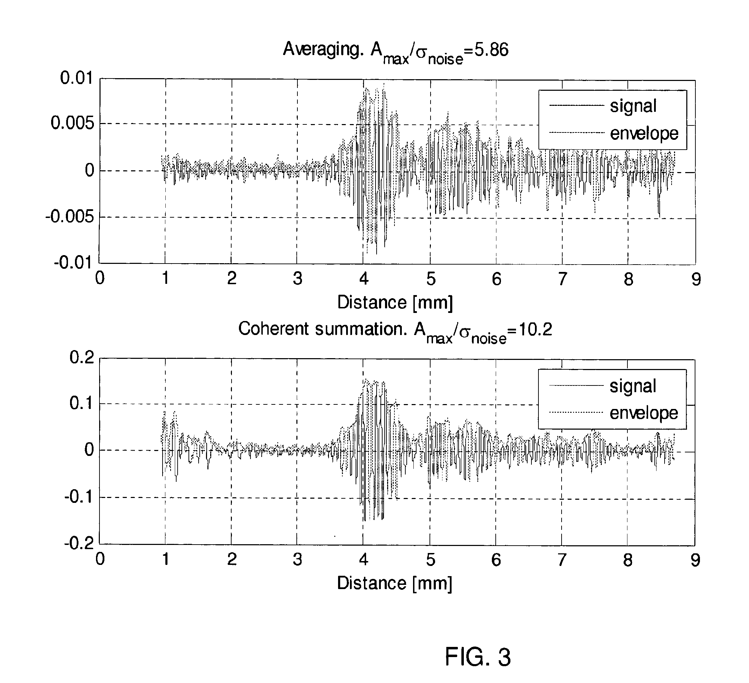

Fig. 2 upper part presents a raw signal and the lower part shows the same after reference subtraction. Fig. 3 upper part shows averaging and gives a ratio of maximum amplitude over noise standard deviation to be 5.86. The signal shown represents results of averaging 11 particular signals. The lower part of Fig. 3 shows coherent summation and manages to achieve a ratio of maximum amplitude over noise standard deviation of 10.2, nearly double the level achieved by averaging.

Fig. 4 presents result of repeated coherent summation of 23 groups (total 11x23=253 processed signals of 256) and convolution with a resonant window.

According to the above considerations, the best improvement of SNR for 11 particular signals is 3.32. It follows from Fig. 3 that the real improvement is 1.74. Specifically, the improvement is the ratio of SNRs after and before processing. SNR after processing is 10.2, and SNR before is 5.96. SNR after processing is greater.

Figures 5A - 8 are as discussed above and illustrate estimated and real distance results for distances of 0.19mm or 0.69mm. Fig. 5A shows the raw signal and Fig. 5B shows the corresponding analytical signal. Fig. 6 shows the curve fitting to find the point at which the primary echo component appears. Fig. 7 illustrates the error function and Fig. 8 illustrates the full phase-distance curve from which local slopes can be obtained.

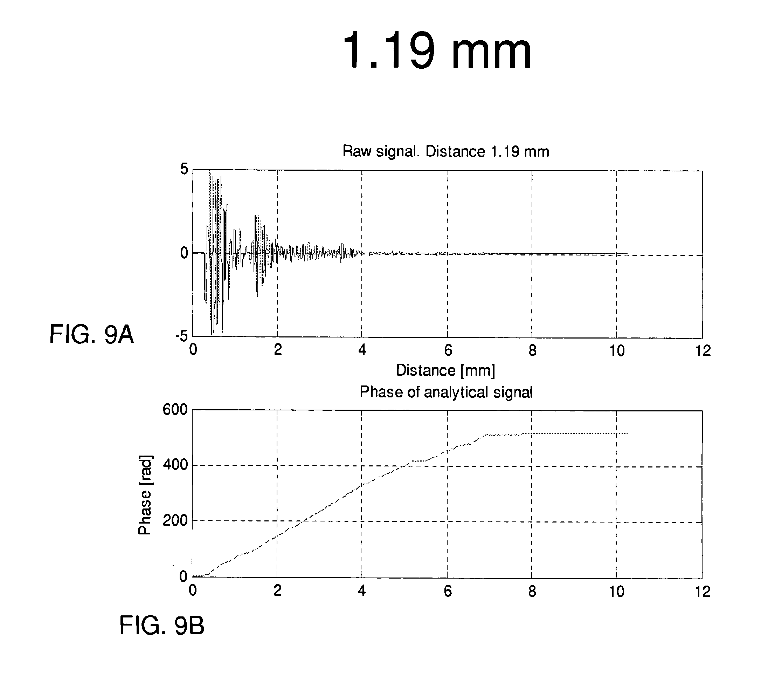

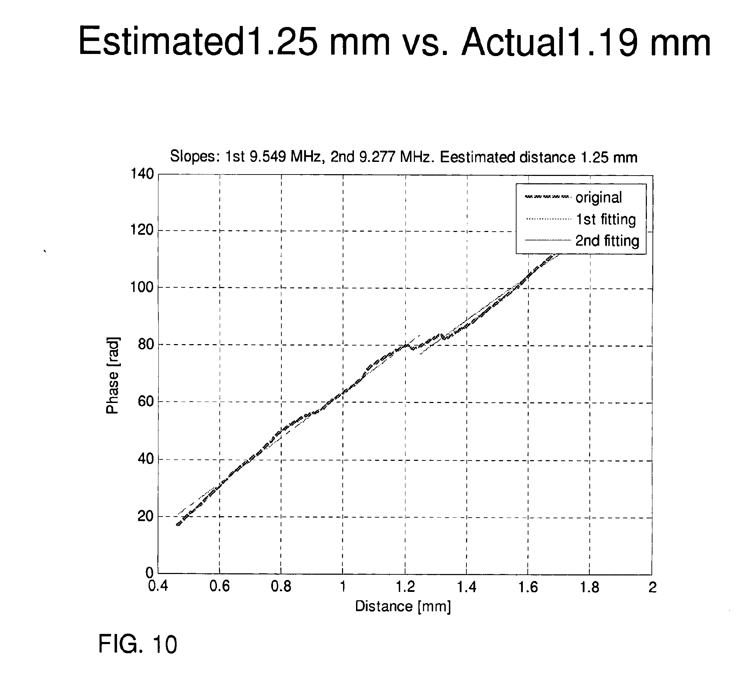

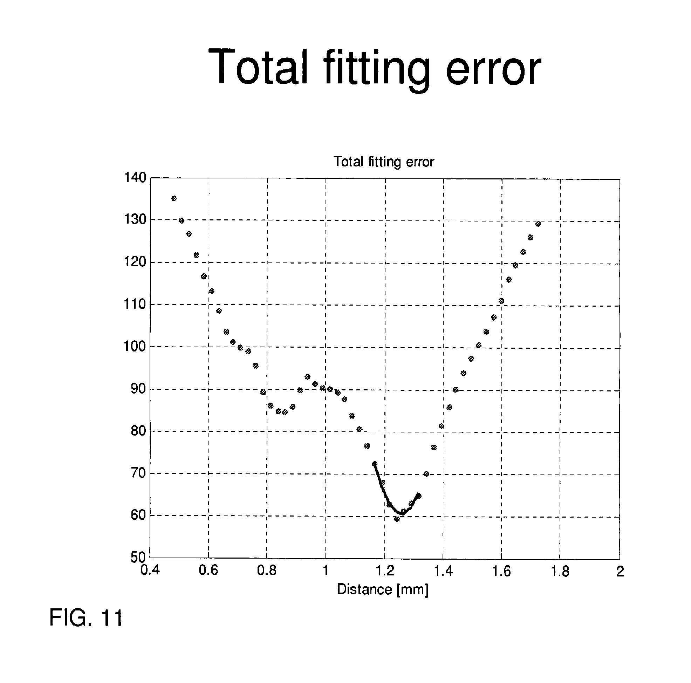

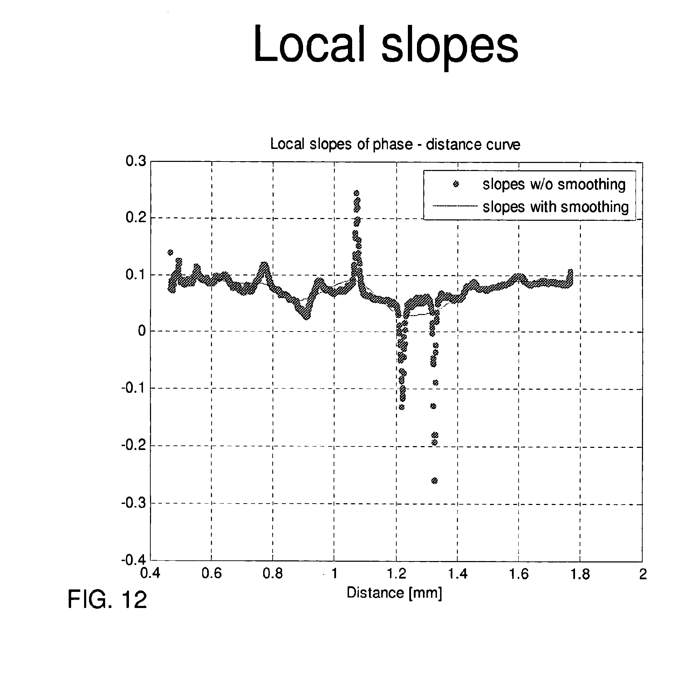

Figures 9A - 12 illustrate corresponding estimated and real distance data for a distance of 1.19mm. Fig. 9A shows the raw signal and Fig. 9B shows the corresponding analytical signal. Fig. 10 shows the fitting. Fig. 11 illustrates the error function and Fig. 12 illustrates the full phase-distance curve.

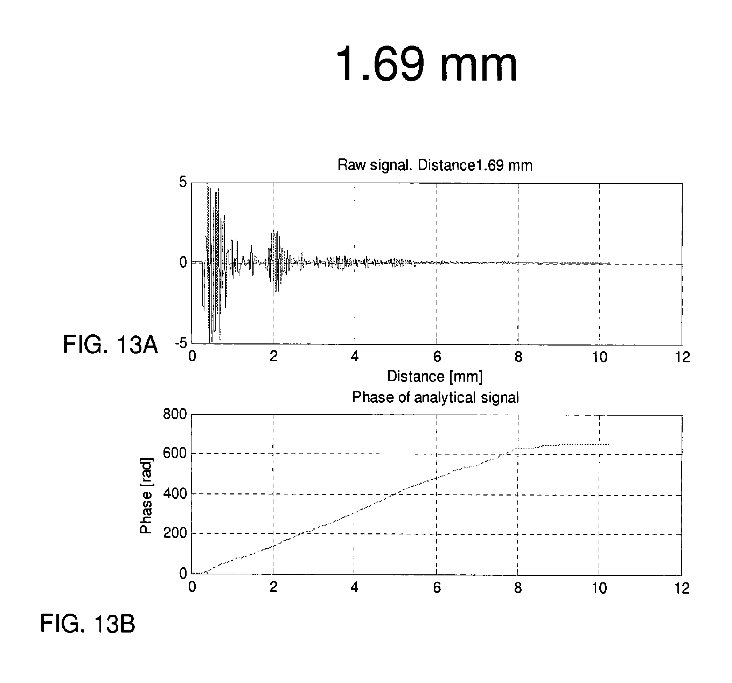

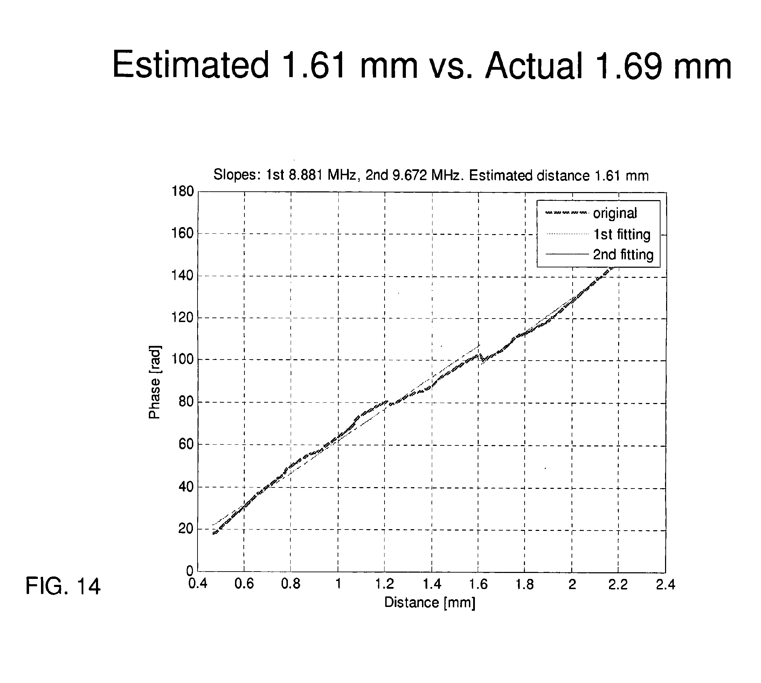

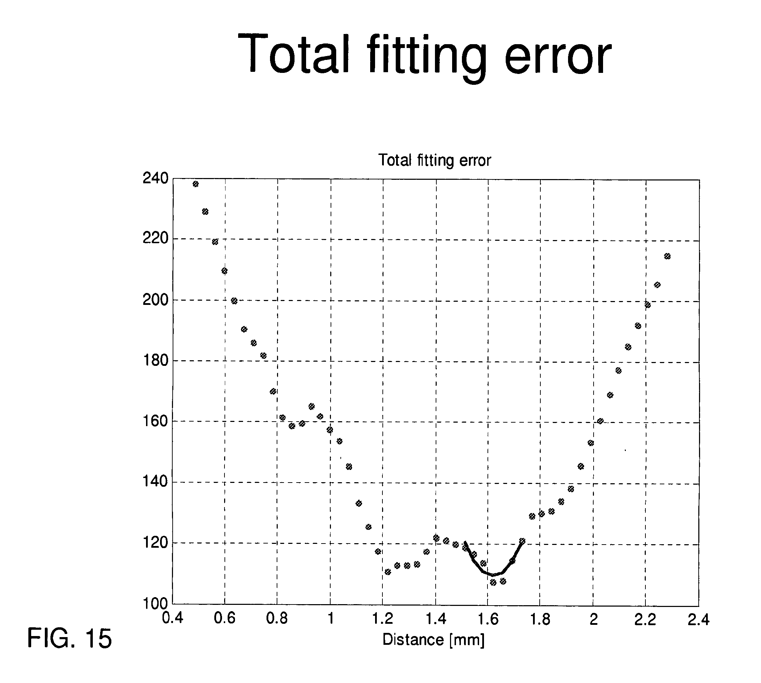

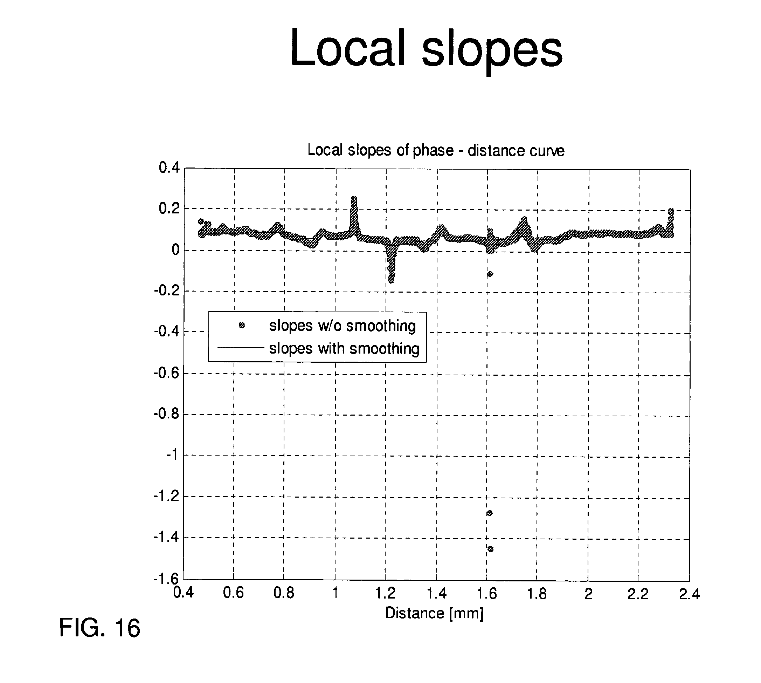

Figs 13A - 16 illustrate results for a distance of 1.69mm. Fig. 13A shows the raw signal and Fig. 13B shows the corresponding analytical signal. Fig. 14 shows the fitting. Fig. 15 illustrates the error function and Fig. 16 illustrates the full phase-distance curve.

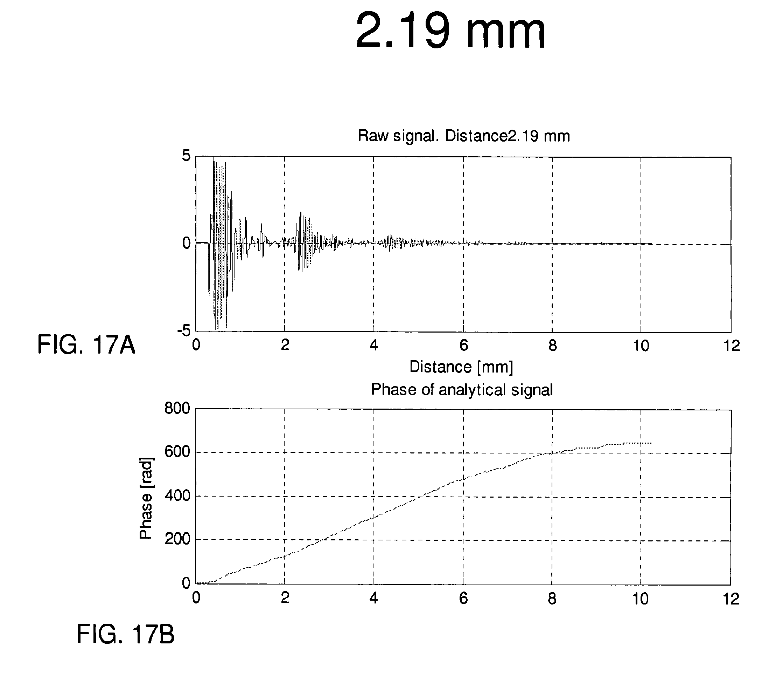

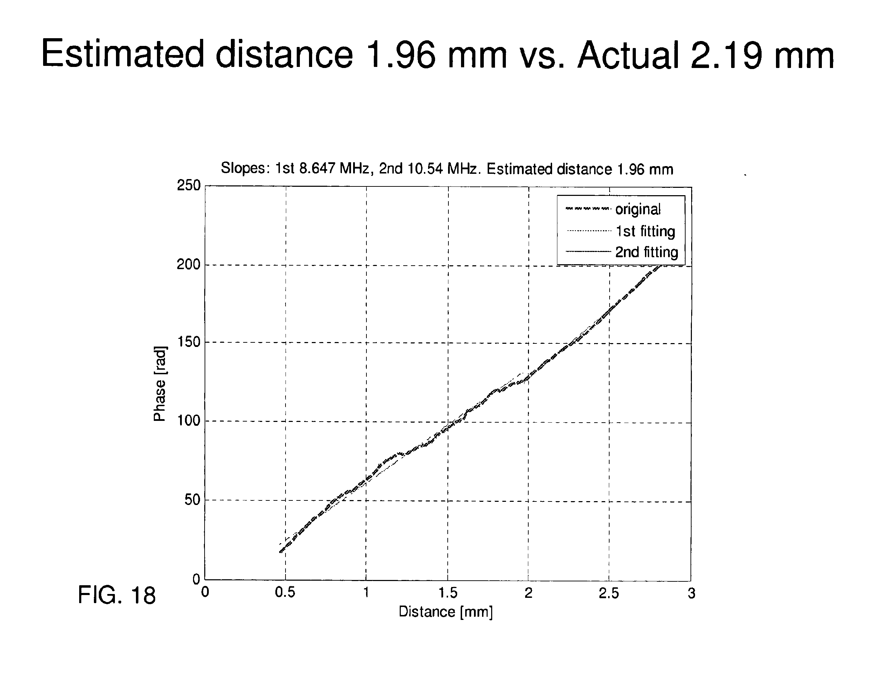

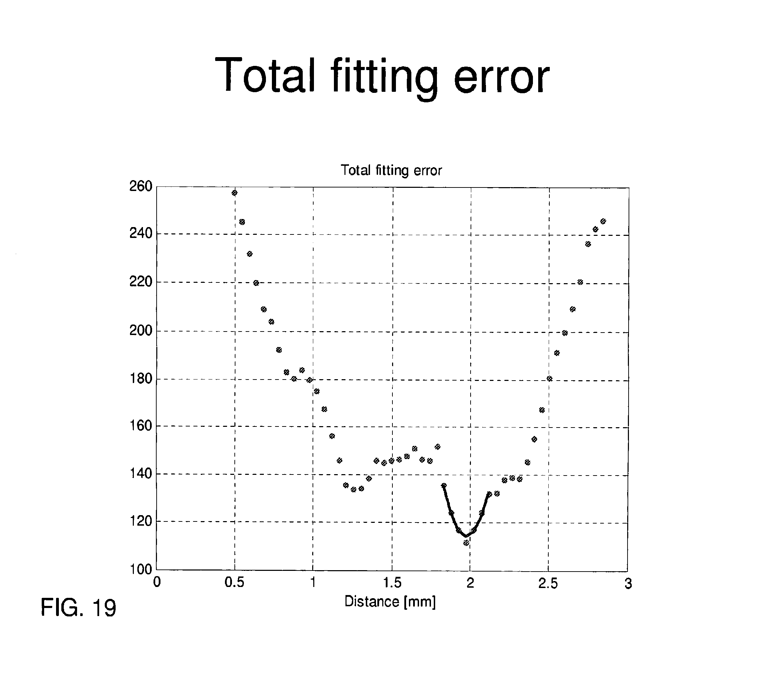

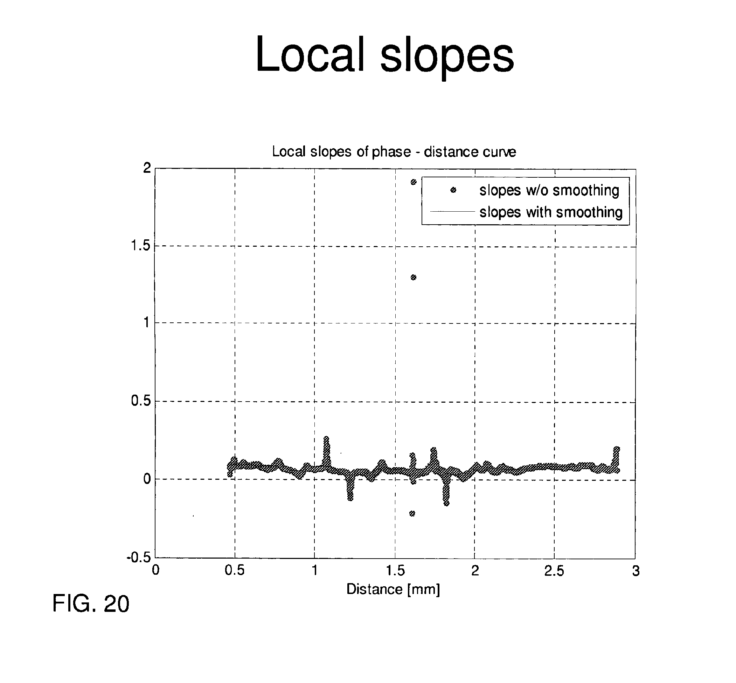

Figs 17 - 20 illustrate results for a distance of 2.19mm. Fig. 17A shows the raw signal and Fig. 17B shows the corresponding analytical signal. Fig. 18 shows the fitting. Fig. 19 illustrates the error function and Fig. 20 illustrates the full phase-distance curve.

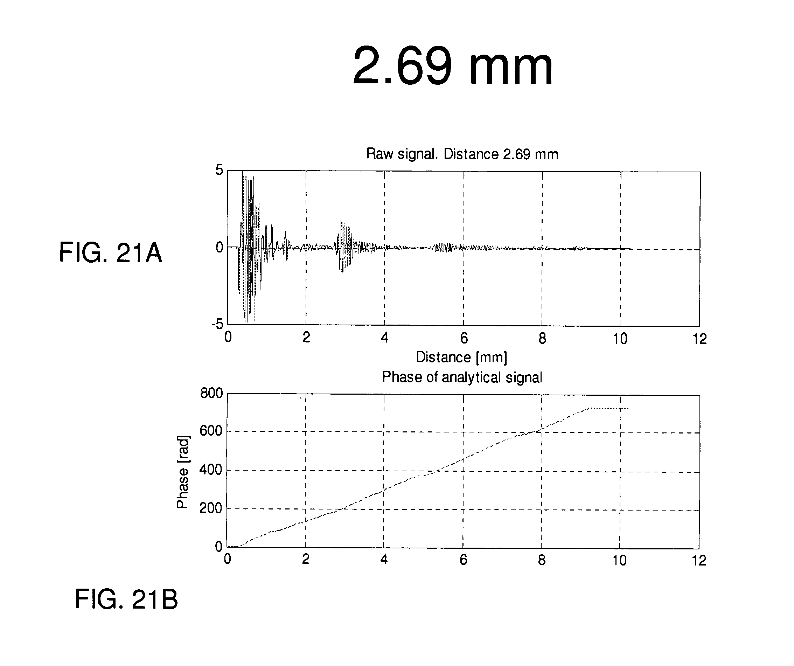

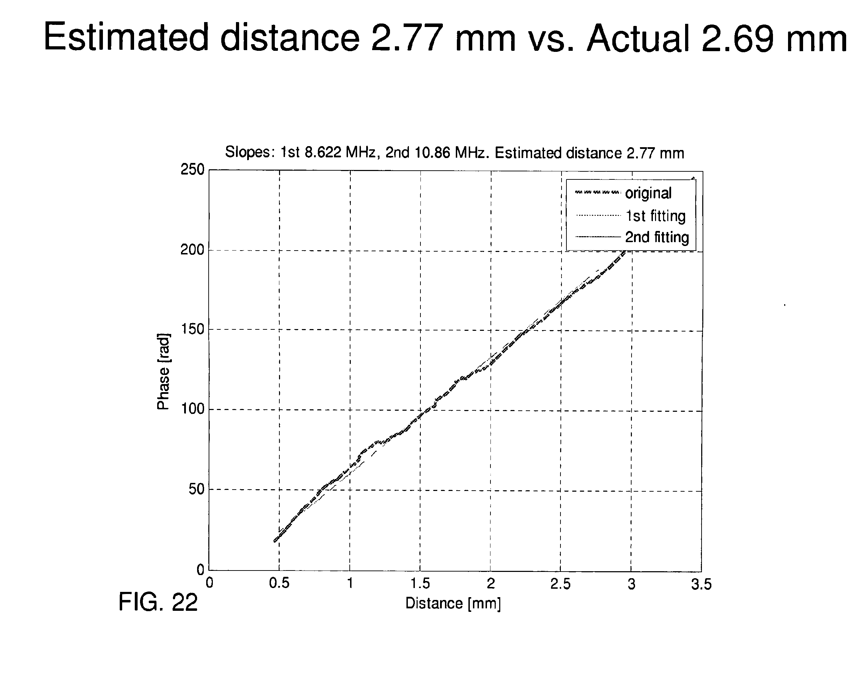

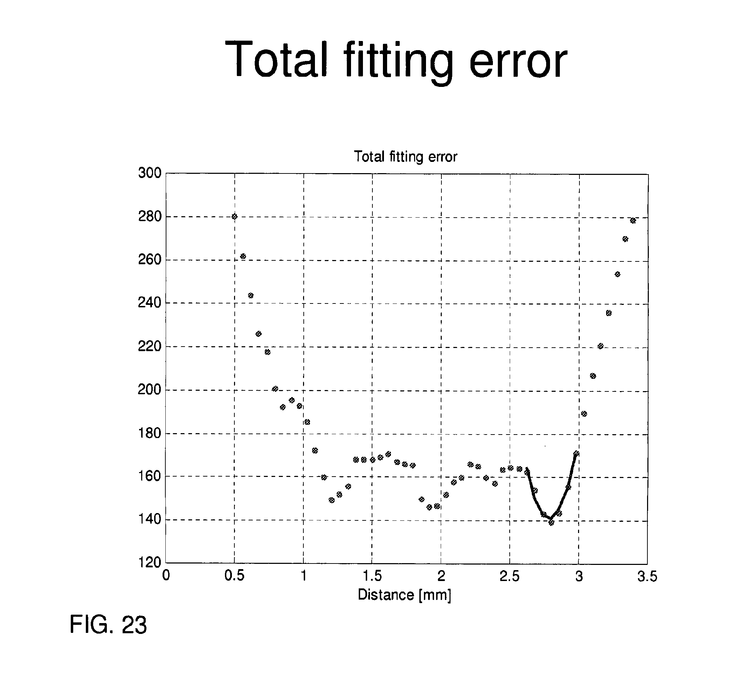

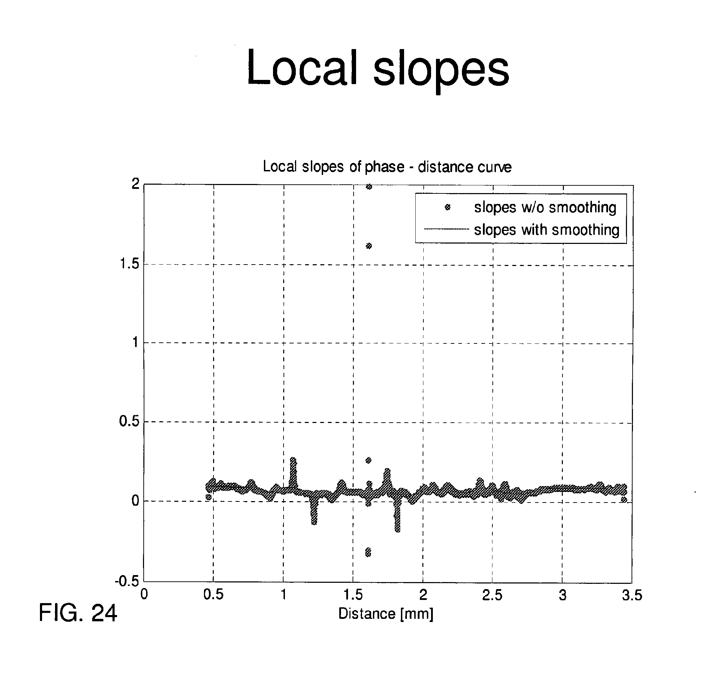

Figs 21A - 24 illustrate results for a distance of 2.69mm. Fig. 21A shows the raw signal and Fig. 21B shows the corresponding analytical signal. Fig. 22 shows the fitting. Fig. 23 illustrates the error function and Fig. 24 illustrates the full phase-distance curve.

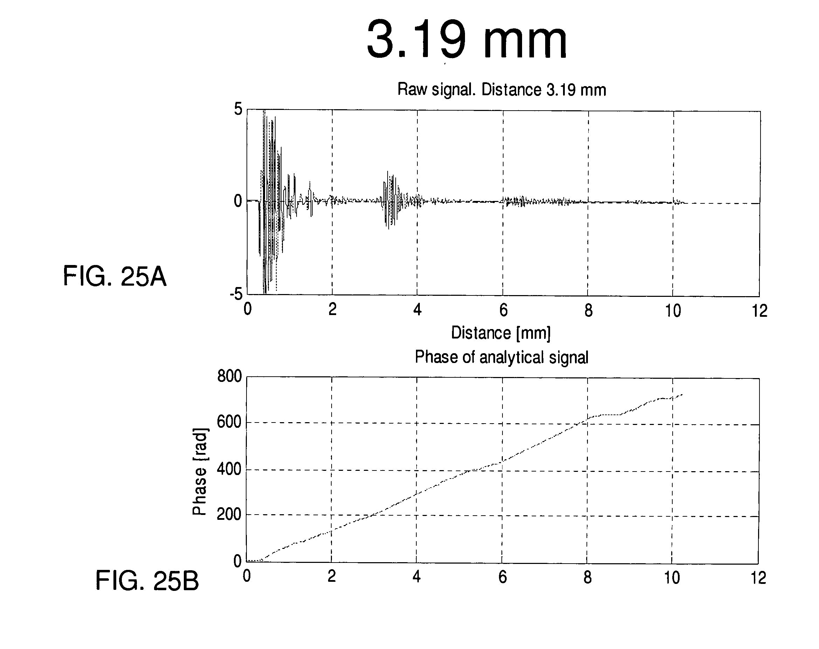

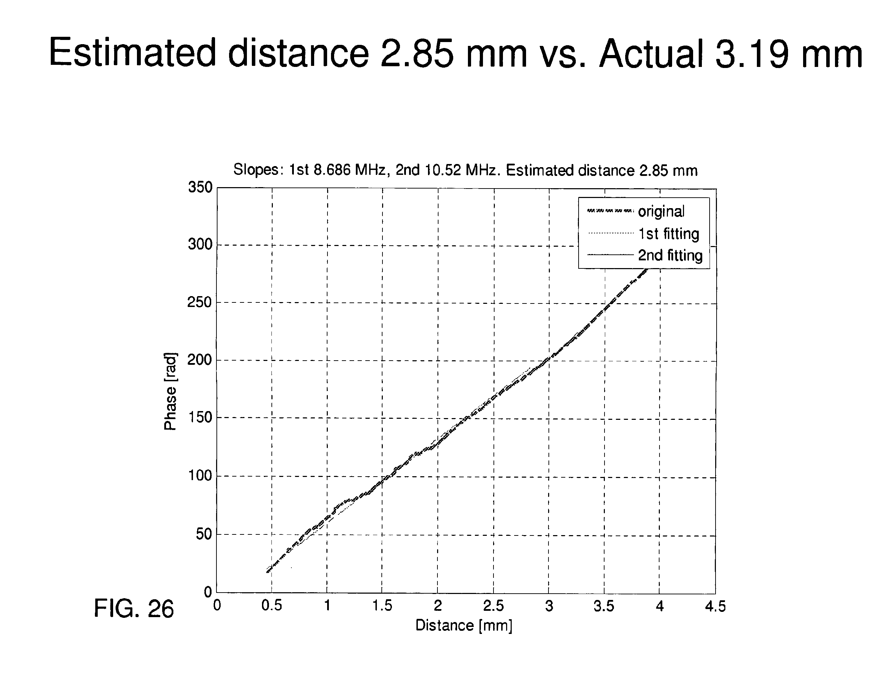

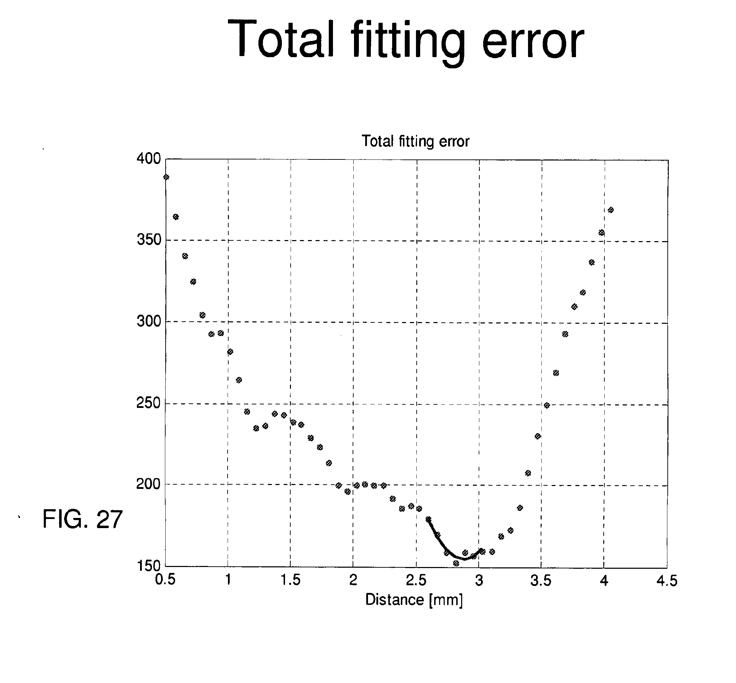

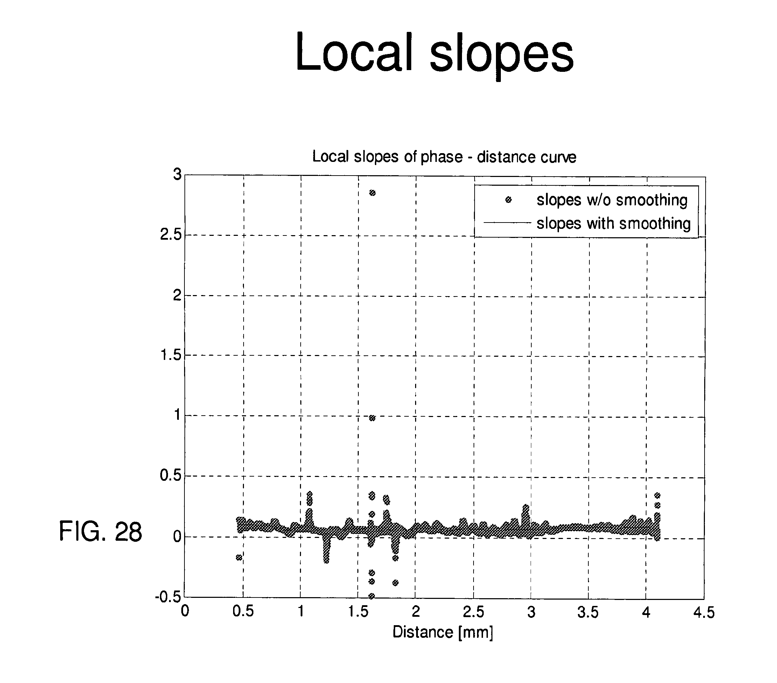

Figs 25A - 28 illustrate results for a distance of 3.19mm. Fig. 25A shows the raw signal and Fig. 25B shows the corresponding analytical signal. Fig. 26 shows the fitting. Fig. 27 illustrates the error function and Fig. 28 illustrates the full phase-distance curve.

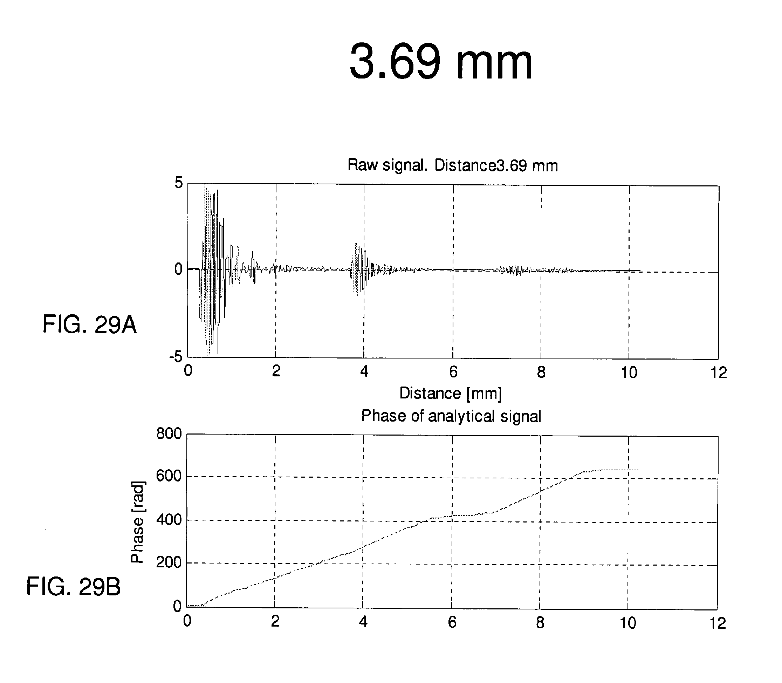

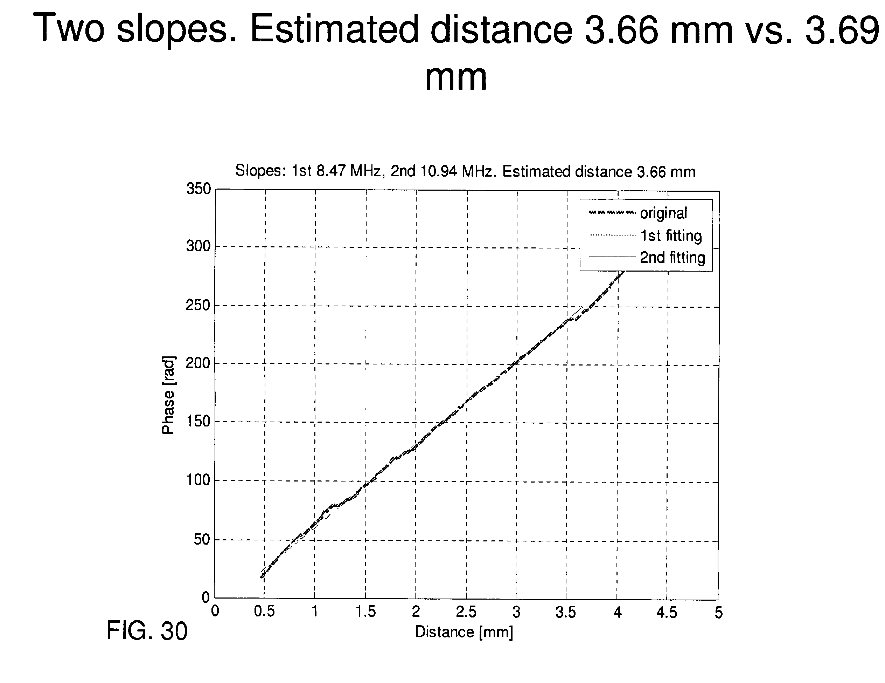

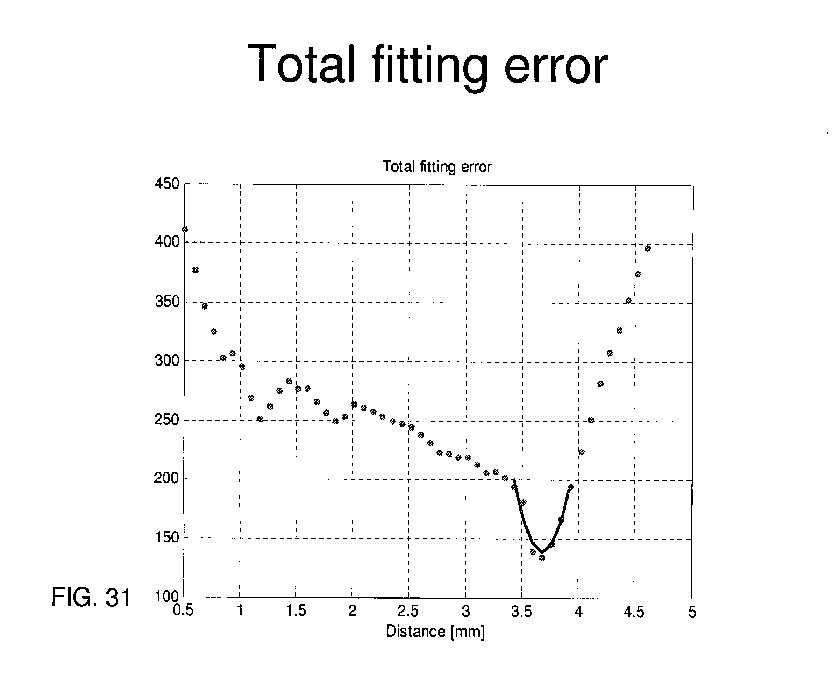

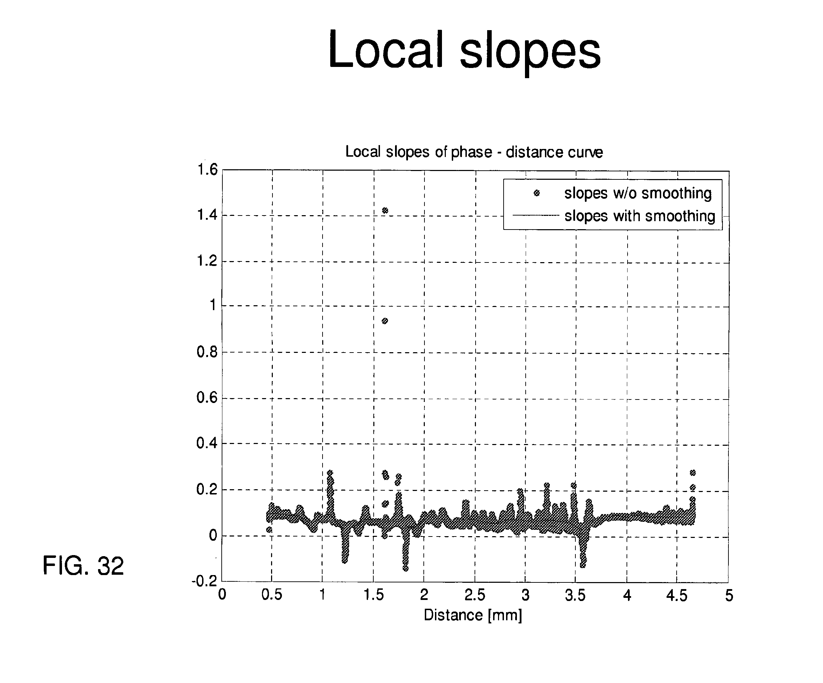

Figs 29A - 32 illustrate results for 3.69mm. Fig. 29A shows the raw signal and Fig. 29B shows the corresponding analytical signal. Fig. 30 shows the fitting. Fig. 31 illustrates the error function and Fig. 32 illustrates the full phase-distance curve.

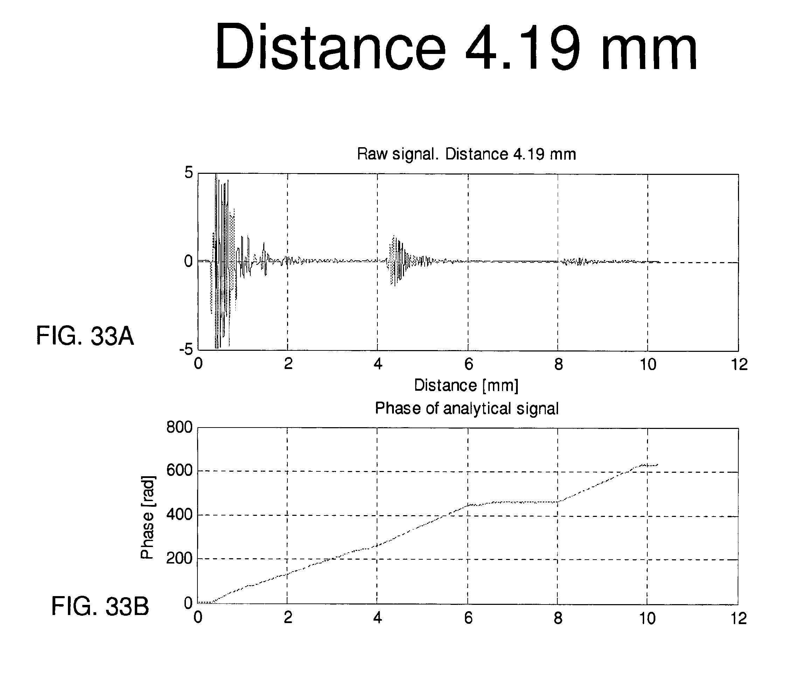

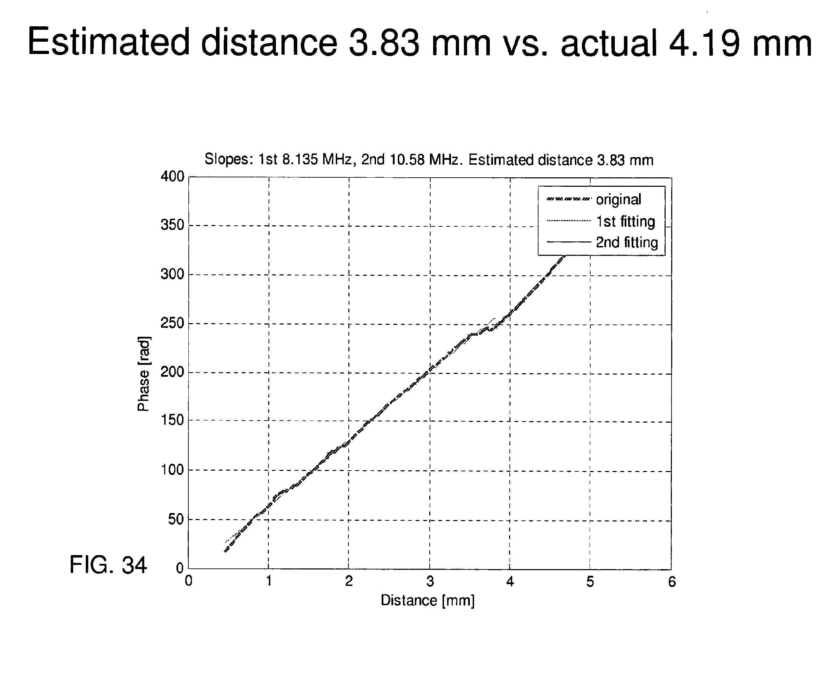

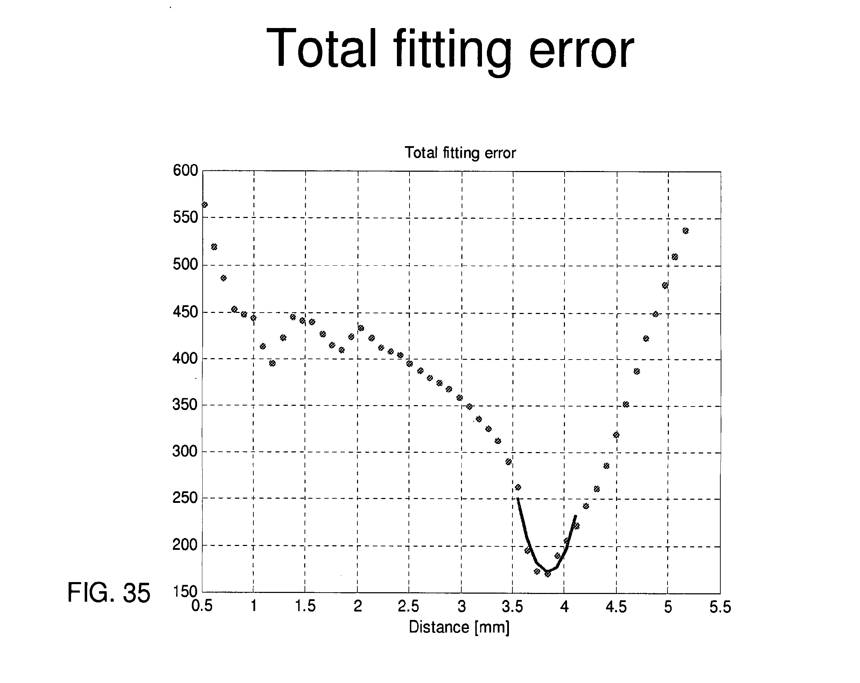

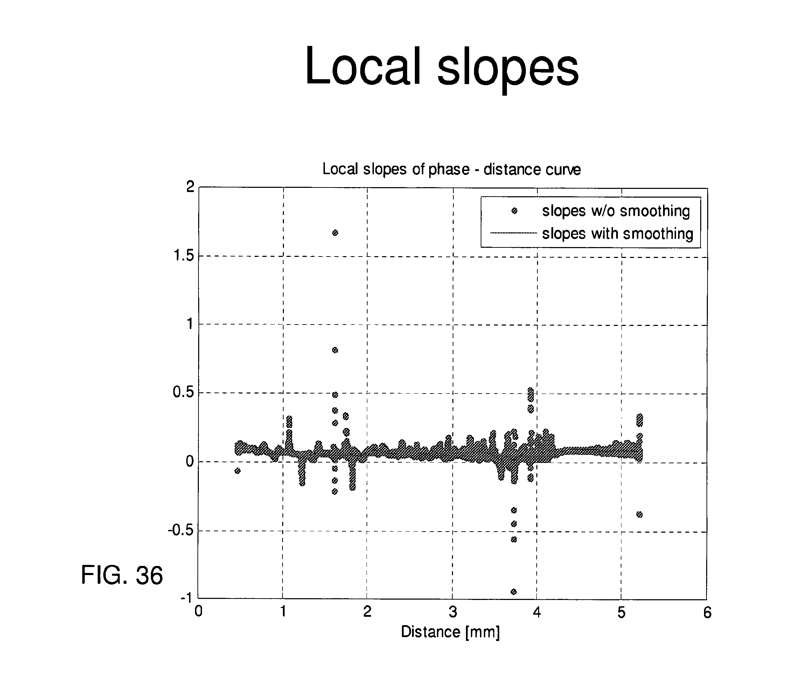

Figs 33A - 36 illustrate results for a distance of 4.19mm. Fig. 33A shows the raw signal and Fig. 33B shows the corresponding analytical signal. Fig. 34 shows the fitting. Fig. 35 illustrates the error function and Fig. 36 illustrates the full phase-distance curve.

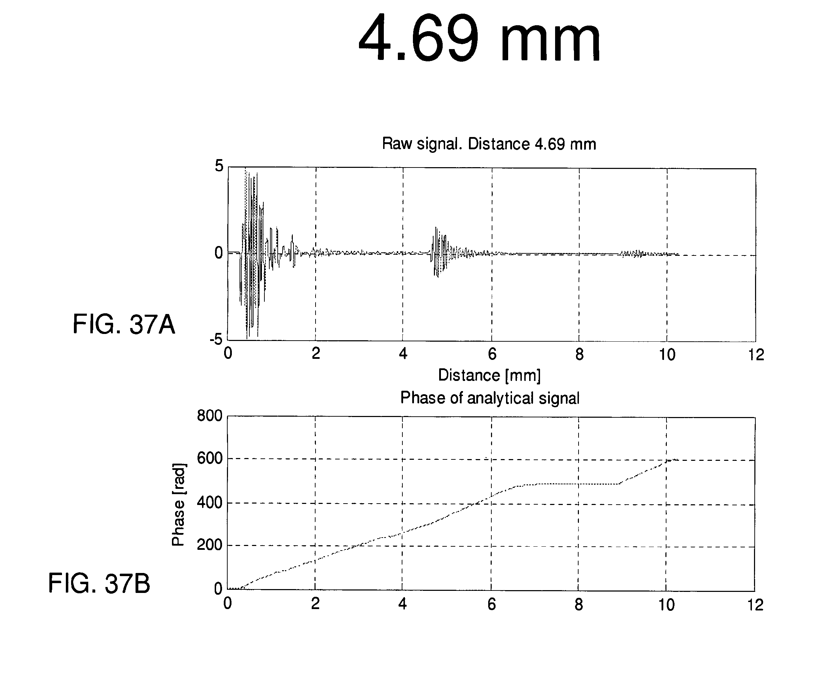

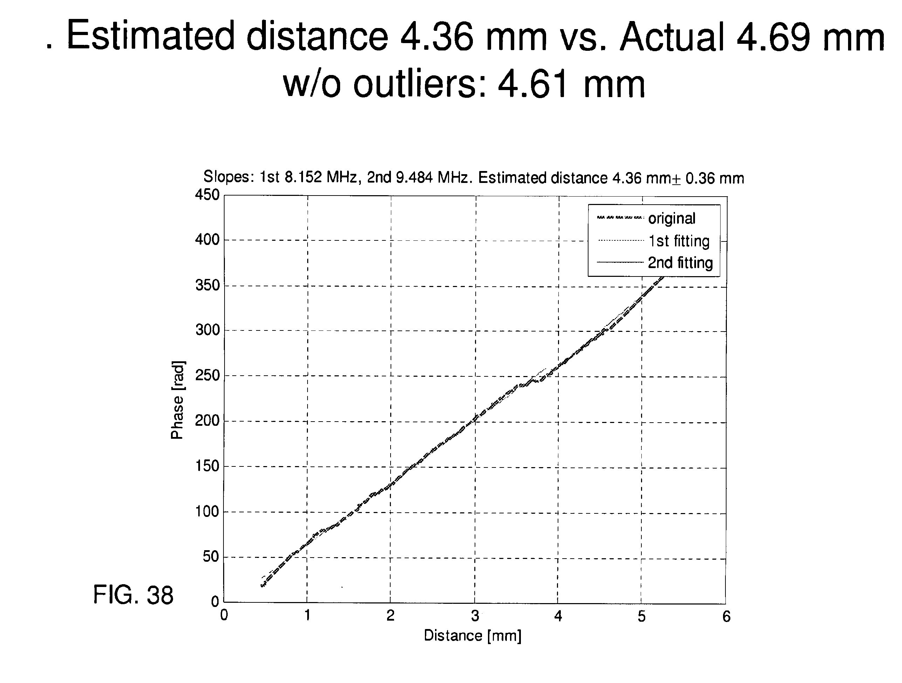

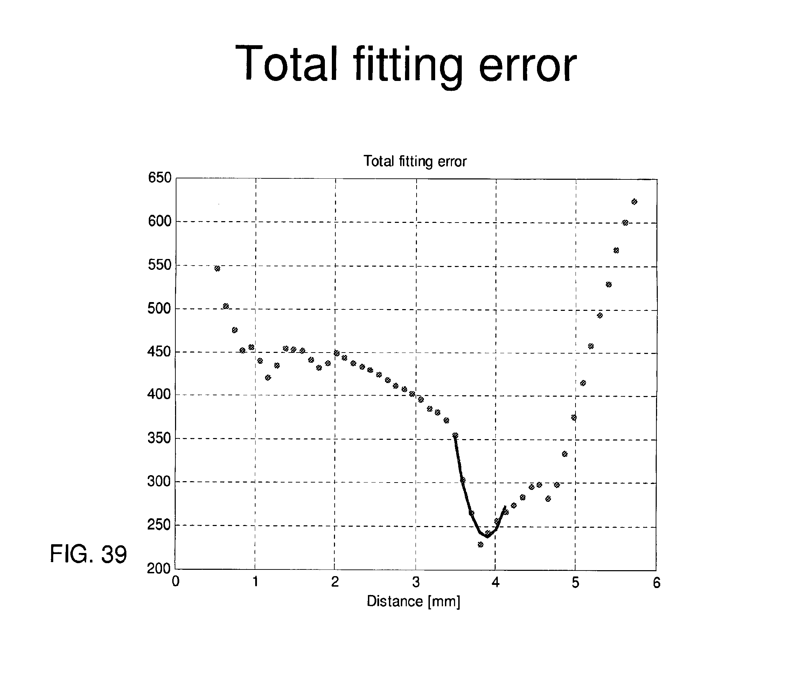

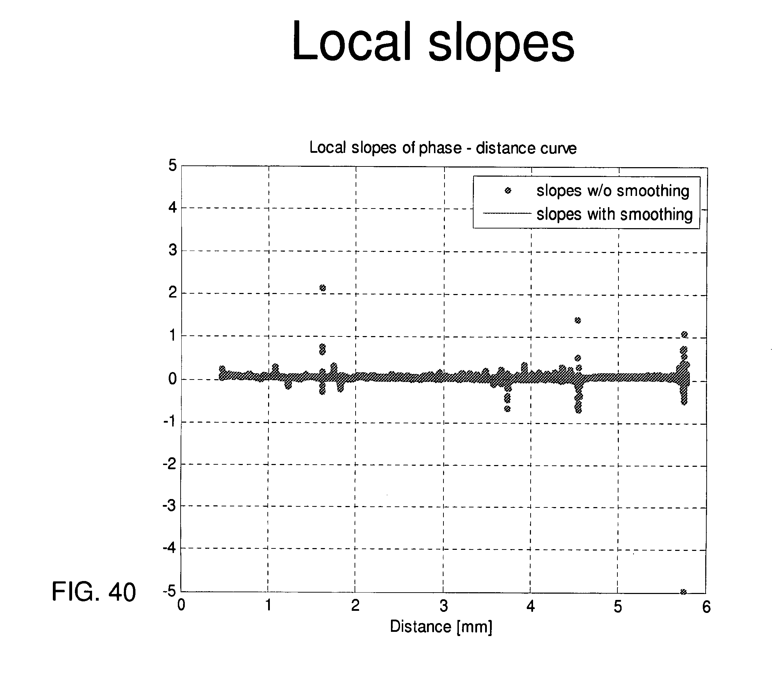

Figs 37A - 40 illustrate results for a distance of 4.69mm. Fig. 37A shows the raw signal and Fig. 37B shows the corresponding analytical signal. Fig. 38 shows the fitting. Fig. 39 illustrates the error function and Fig. 40 illustrates the full phase-distance curve.

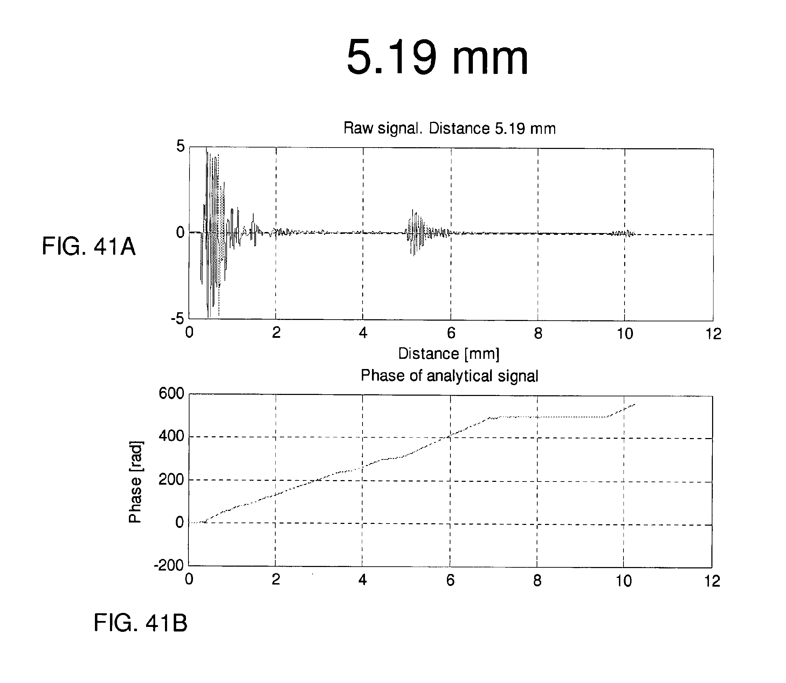

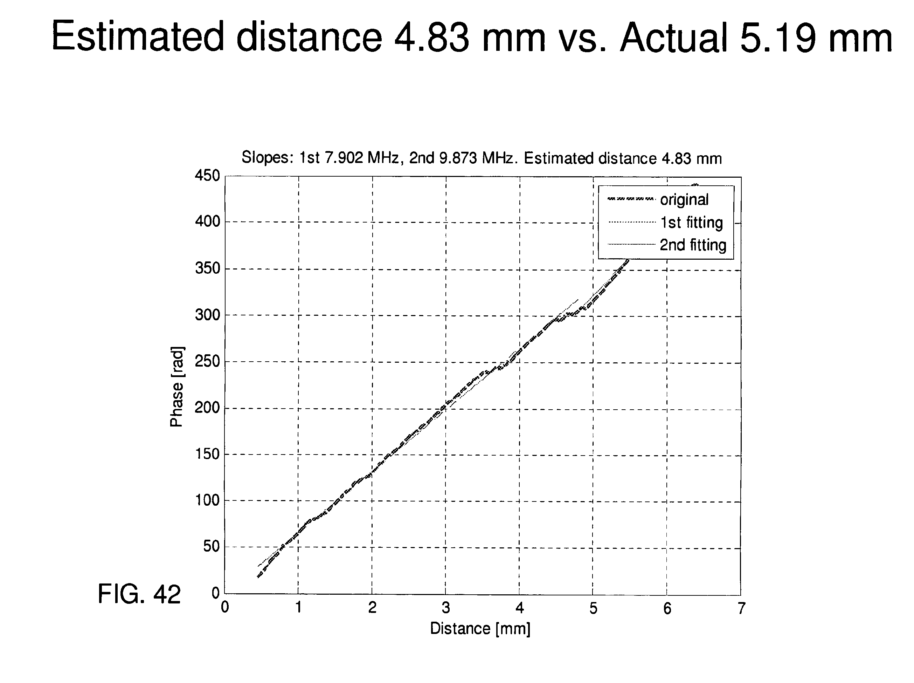

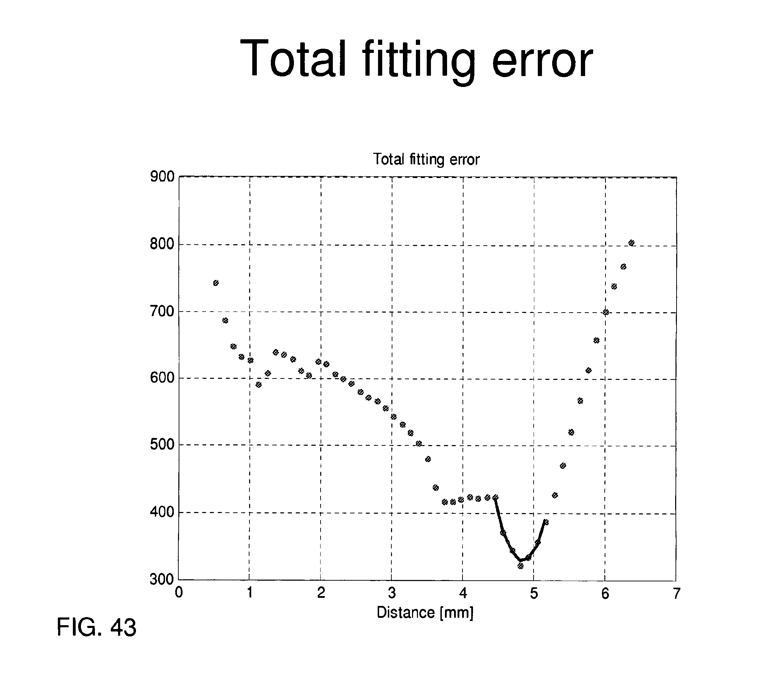

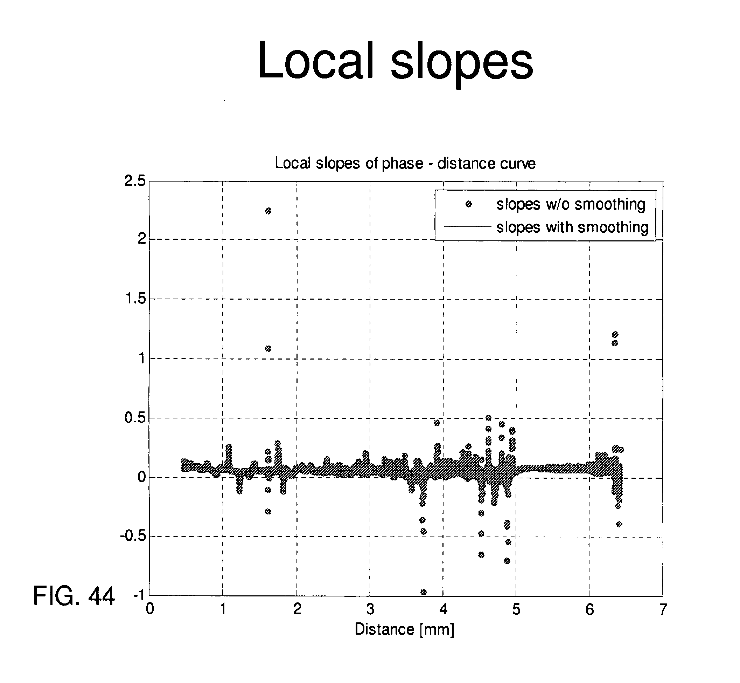

Figs 41A - 44 illustrate the results for a distance of 5.19mm. Fig. 41A shows the raw signal and Fig. 41B shows the corresponding analytical signal. Fig. 42 shows the fitting. Fig. 43 illustrates the error function and Fig. 44 illustrates the full phase-distance curve.

Each estimation is average on 6 sets of 5 times averaged data. Except for one point (4.69 mm), standard deviation is not significant. With outliers excluded, estimation in this point gives 4.61 mm. Absolute RMS error is 0.24 mm.

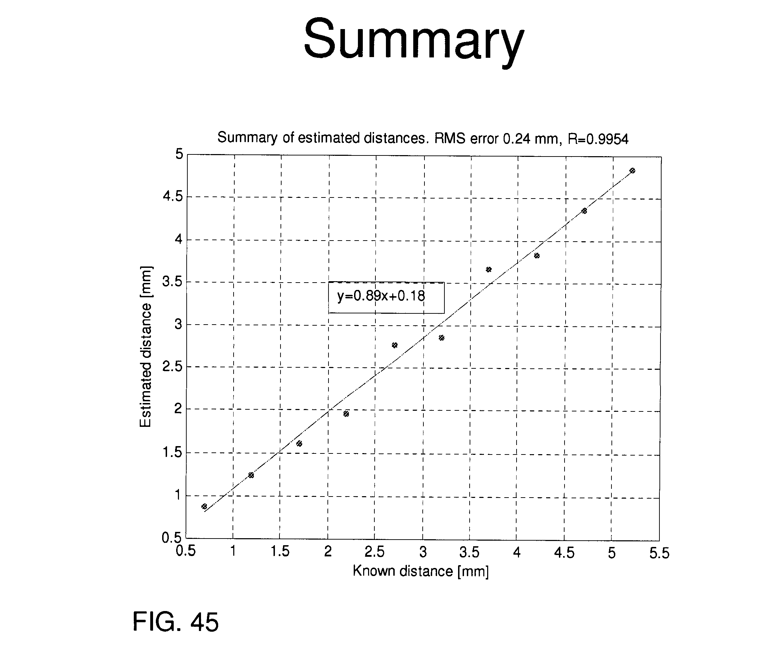

Referring now to Fig. 45, a correlation curve is obtained by fitting measured results to actual distances. A straight line is fitted to the data points, with slope 0.89 and intercept 0.18 mm. Correlation of known and estimated distances is high, and the observed correlation coefficient is 0.9954.

General description of the problem and requirements

The procedure is now considered in more detail. It is required to estimate distance from a transceiver surface to artery wall. The range of interest lies between 1...5 mm. Allowed error is 0.2 mm. Processing time is not intended to exceed 2s.

Features and difficulties

As mentioned, there is uncertainty in mutual orientation of and artery wall, known as angular uncertainty.

The reflected signal is typically small. That is, first, it is corrupted by noise in general and, second, is distorted by a residual excitation signal, otherwise known as ringing. As mentioned, the ringing can produce second order effects such as echoes of the ringing. The reflected signal is a superposition of multiple similar signals each produced by its own source the source. That is, the shape of reflected signal may vary depending on the angular position of the transceiver.

Within the same *in vivo* experiment, it is possible that a given transceiver surface may be polluted with coagulated blood, causing changes in the characteristic frequency.

Fortunately, in general, applied transceivers are narrow band devices and reflected signals are close to harmonic, however, with variable amplitude. This allows for efficient signal isolation.

Data Averaging

Data are built as a matrix, whose columns represent a reaction wherein a single record maps to a single pulse. Then, columns of the records are divided into groups on which averaging is carried out. Following this, each column is processed on the basis of the averaged groups and overall results are averaged. In the matrix, row size may represent the total number of points recorded in a single echo response, and the column numbers may represent the total number of echo responses which was recorded.

As an example of the above, in *in vivo* experiments which were carried out, datasets were obtained. Each dataset consisted of 1000 records, each having about 12000 samples, and corresponding to a duration of about 0.15 $\mu$s. The PRF was 400 Hz, therefore total duration of the experiment was 2.5 s. Sometimes only the first 256 records were used for processing. The first 1000 samples (about 0.95 mm) were excluded from processing. Data length was restricted to $2^{13}$=8192 samples (about 8 mm) to accelerate Fourier transforms. The best case occurs when the number of samples is a power of 2. However, FFT performance it is also good if it is the product of several primes. Optional fine tuning of data length is also carried out.

Reference subtraction

At small distances the entire signal is a superposition of the residual transceiver signal, the free reaction, and the reflected transceiver signal. An apparent solution would be to subtract the pure excitation signal without reflections from the superposition to obtain the reflection, but this fails to take into account noise or second order effects.

Thus in an embodiment, a reference signal is obtained by averaging of several records. Presumably, due to instabilities, phases of reflected signals are randomly different from each other, and averaging weakens this component. Contrary, the excitation pulse is taken as stable so averaging does not cause any deterioration. Thus, in principle the unstable component is more or less efficiently eliminated from the processed signal.

Noise level

Noise levels may be estimated from a remote part of a record where reflections are not expected.

Coherent Summation

Having identified the most relevant windows, the procedure then identifies the primary echo based on coherent summation. Consider a set of signals, which are expected to be similar to each other. Ideally, all signals would be the same, except for the noise component. Thus summation of $N$ such signals increases the entire *signal N* times while noise increases only $\sqrt{N}$ times. As a result, the SNR improvement is $\sqrt{N}$ times. In practice, however, due to random phase shifts the described procedure becomes less efficient. This can be solved by means of a Hilbert transform producing a complex analytic signal, in which each signal can be assigned a phase, which, for a true harmonic signal would be the true phase. Thus, phase is defined conventionally as for a usual complex number:

$$\varphi = \arctan \frac{\Im(Hx)}{\Re(Hx)} \qquad (1)$$

Let the $k$-th signal generate Hilbert phases $\alpha^{(k)}$. We wish to find a transform which preserves amplitude and brings all signals of the set to the same phase or, at least, minimizes phase deviation of each signal from an averaged phase $\overline{\alpha}$. Let ..., $z = x + ky$ be the complex analytic signal generated by the Hilbert transform. Thus new signals are created as follows:

$$u = x \cos \gamma - y \sin \gamma, \quad v = x \sin \gamma + y \cos \gamma$$

which may be expressed as,

$$w = z \exp(i\gamma).$$

Such a transform preserves amplitude

$$\sqrt{u^2 + v^2} = \sqrt{x^2 + y^2} = a.$$

We wish to minimize (weighted) error

$$\varepsilon = \sum_k W^{(k)} \left| \frac{z^{(k)} \exp i\gamma_k}{a^{(k)}} - \exp i\overline{\alpha} \right|^2.$$

For simplicity, summation over elements of each signal is omitted. In equivalent form,

$$\varepsilon = 2 \sum_k W^{(k)} - 2\Re \sum_k \frac{W^{(k)}}{a^{(k)}} \left( z^{*(k)} \exp(\overline{\alpha} - i\gamma_k) \right).$$

Since we wish to have the best agreement for signal but not for noise, weights may be chosen proportional to amplitude. Angles providing minimum error for the $k$-th signal are found from the equation

$$\frac{\partial \varepsilon}{\partial \gamma_k} = 2\Re \left( iz^{*(k)} \exp(i\overline{\alpha} - i\gamma_k) \right) = 0.$$

In matrix notation,

$$\mathbf{E}_k = \exp(i\gamma_k) = \frac{\mathbf{z}^{H(k)}\mathbf{e}}{\left\|\mathbf{z}^{H(k)}\mathbf{e}\right\|}, \quad \mathbf{e} = \exp(i\overline{\alpha})$$

$$\tag{2}$$

The denominator provides proper normalization such that entries of the above weights matrix are true phase exponents. The upper index H means matrix Hermit conjugation (or Hermitian conjugation), which is transposition together with complex conjugation. The signal after coherent summation is simply

$$\mathbf{x}_{coh} = \Re(\mathbf{zE})$$

$$\tag{3}$$

A first factor in equation 3 is the analytic signal corresponding to the entire set of signals. A second factor is an auxiliary phase weight matrix, which provides summation with proper phases.

Step-wise algorithm description

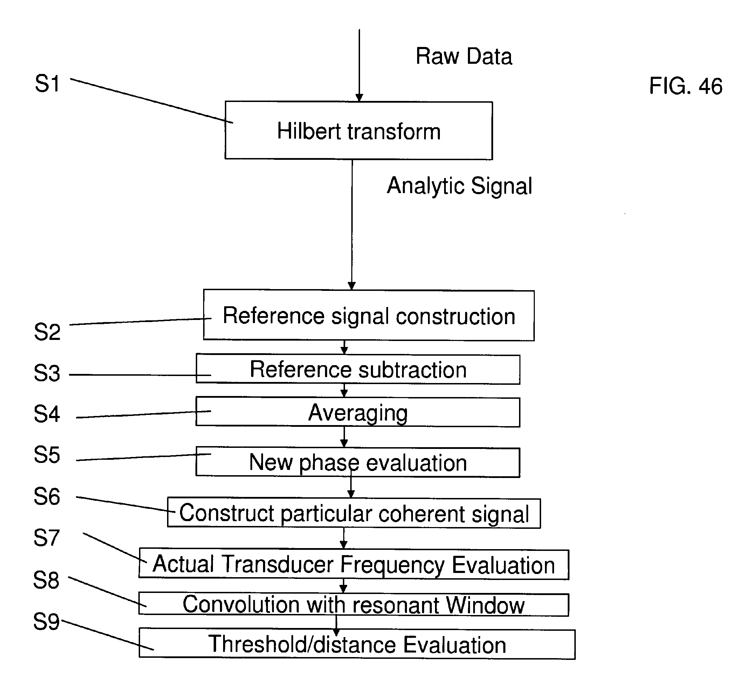

Reference is now made to Fig. 46 which illustrates a possible procedure.
S1 involves carrying out a Hilbert transform on the entire raw data:

$$z \quad = \quad Hx \quad = \quad x \quad + \quad iy$$

to obtain an analytic signal. S 2: Reference signal construction uses

$$z_{ref} \quad = \quad \frac{1}{N}\sum_{k=1}^{N} z^{(k)}$$

S3: Reference subtraction. In an embodiment, individual subtraction is used. That is to say, for each set of data, the reference signal is multiplied by an individual scaling factor which is close to 1. The scaling factor is evaluated using the initial portion of data (below 1 mm). Individual scaling factors provide the least RMS error being applied to a particular signal, but to a portion, which is not involved in further processing. In the present embodiments, these values are propagated to the remaining portion of the data for more efficient subtraction of the excitation signal.

$$z \quad \rightarrow \quad z \quad - \quad z_{ref}$$

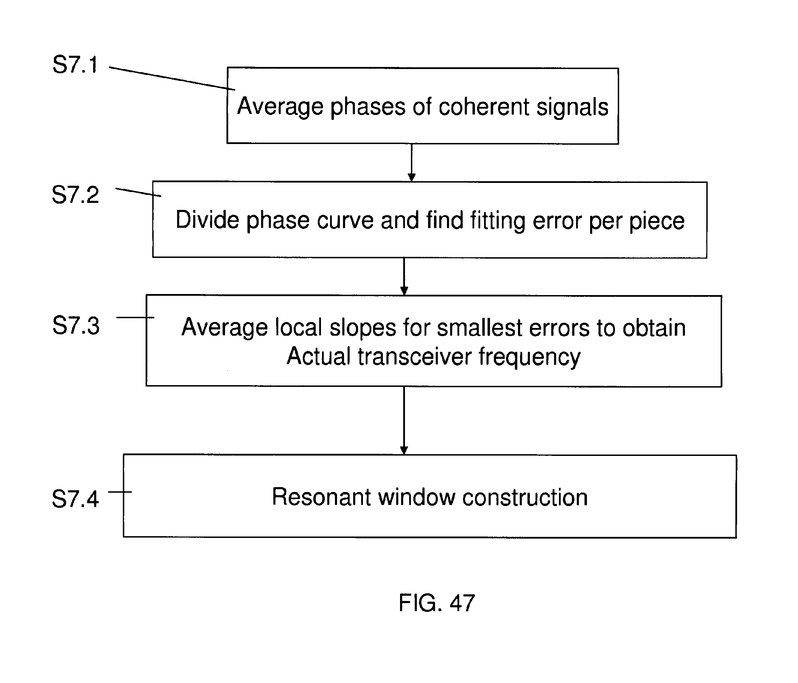

S4: Averaging. Each group of columns is averaged. Members of the group participate in the coherent summation procedure. In an embodiment, an optimal arrangement of groups is used. In this arrangement, within each group, phases are closer to each other.
S5: carrying out a phase evaluation, again based on formula (1).
S6: Construction of particular coherent signal. See formulae (2) and (3).
S7: Actual transceiver frequency evaluation. S8 is shown in greater detail in Fig. 47.
In S7.1, phases of all particular coherent signals are averaged.
In S7.2, the entire phase curve is divided into small pieces (about 100 samples), and for each piece a local strip and a normalized linear fitting error are evaluated. Optionally, local slopes are obtained on overlapping pieces. An instantaneous frequency may be found at each point. Alternatively, instantaneous frequencies may be obtained by usage of a formally exact definition of frequency as a phase time derivative. A Hilbert transform of the derivative may be involved.
In S7.3, for some of the smallest errors (about 10), corresponding local slopes are averaged, to give the actual transceiver frequency.
In S7.4, the actual transceiver frequency is used for resonant window construction. Namely,

$$w_{res}(t) = w(t)\exp(2\pi i f t)$$

A first factor in the above is a standard windowing factor (Hanning, Gauss, Kaiser, etc.). A size of the window may correspond to 2 periods.

Returning now to Fig. 46 and in S8: Convolution with a resonant window is shown. An absolute value of the convolved complex signal (amplitude, or envelope) is used for further analysis.

S9: Thresholds and distance evaluation. It must be emphasized that there is no rigorous rule to choose a threshold for a reflected signal appearance because its shape may change even within the same experiment. Besides, the origin of a transceiver transient reaction may be inherently small and therefore to all intents and purposes are invisible. However, one can set an empirically based value of a threshold and consider reflection appearance as the point at which a signal firstly falls below the threshold, starting from its maximum. Since signal growth usually occurs rapidly, the result must not be sensitive to the threshold variations or uncertainty. In the current version of the algorithm, two alternate threshold estimations are used:

1. Noise level. A remote section of the data where reflections are not expected is used to estimate noise level. The threshold may then be set as

$$T_1 = k_1 \langle s_{noise\,only} \rangle$$

Since the final signal is amplitude (envelope), noise level is estimated as its mean.

2. Peak height. A threshold is

$$T_2 = k_2 s_{max}$$

This second definition is used when the noise level is found to be too high. Sometimes extremely stretched signals are observed. Dimensionless factors in these formulas are established based on visual analysis of the processed signal and may be subjected to fine tuning.

It is possible that reflected signals start at a relatively high level and do not fall below a threshold. Such would be an indication that the origin is closer than 0.95 mm.

Examples

Let us define SNR as a ratio of the largest amplitude of a reflected signal to SD evaluated in the region of pure noise:

$$SNR = \frac{\max(A)}{\sigma_{noise}}$$

Considering again Figs. 1 to 3, Fig. 1 presents a raw signal and the same after reference subtraction. Fig. 2 presents results of just averaging 11 particular signals vs. coherent summation. Fig. 3 presents the result of repeated coherent summation of 23 groups (total 11x23=253 processed signals of 256) and convolution with a resonant window.

According to the above considerations, the best improvement of SNR for 11 particular signals is 3.32. It follows from Fig. 3 that the real improvement is 1.74 based on the SNR values shown and as discussed above.

Fourier Component Approach

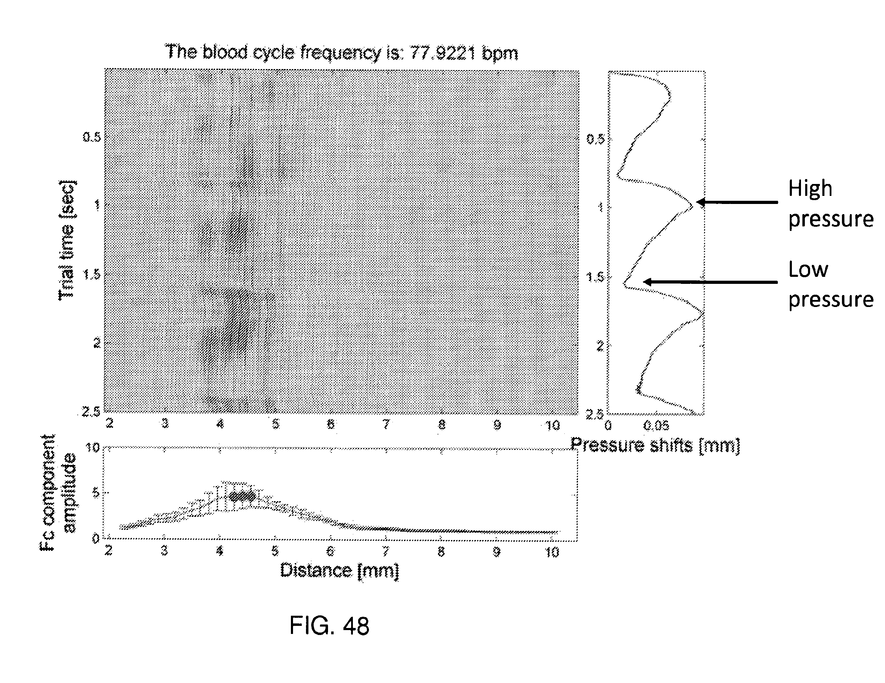

Reference is now made to Figs. 48 - 51 which illustrate an approach based on grouping of samples according to relationships between the echo signals, body frequencies such as pulse or breathing, and the original excitation. The relative position of tissues within the body changes over short times due to breathing and blood pulsation. Ultrasonic echoes from tissues obey such a periodicity whereas ringing artifacts and other types of noises do not. This is specifically true for ultrasonic echoes measured intravascularly. Consequently, the existence of such periodicity can be utilized to separate between tissue reflection and other noise sources in echoes with low signal to noise ratio.

In order to use this information, one must first estimate the periodicity of the echo reflections over consecutive measurements. This periodicity reflects both the movement of the catheter as well as the movement of the tissue.

Several ways can be used to identify the movement profiles of tissue in echo signals. One way is to obtain segments of the echo signals in which a strong reflection is observed. This method is described as follows. Figure 48 (upper left part) shows the echo response of consecutive trials as a function of distance. A clear periodic change in the echo response from the tissue is observed. Since a narrow band transceiver is used, it is possible to quantify the reflection intensity by calculating the Fourier component of the central transceiver frequency Fc in consecutive time windows (Fi) of duration D and overlap OL:

$$F_i = \sqrt{\left(\int \sin(2\pi F_c t) X(t) dt\right)^2 + \left(\int \cos(2\pi F_c t) X(t) dt\right)^2}$$

The response profile in the time domain is illustrated in the lower part of Fig. 48. From the profile, ND windows with the highest values of the central frequency Fourier component are chosen for cross-correlation analysis. The relevant windows are marked by red circles in the lower part of Fig. 48. Using cross correlation analysis between consecutive trials, it is possible to identify the shifts in distance between the tissue and the catheter, as illustrated in the right part of figure 48. Once the movement profile of the tissue is obtained for many tissues samples, this movement profiles can be used to estimate the characteristic frequencies related to breathing and blood pulsation.

Then, the characteristic frequency profiles due to tissue movement are used to separate between the characteristic frequencies and ringing artifacts and other noise signals. In greater detail, owing to the blood pressure, the catheter moves at similar frequencies to the blood pressure, as illustrated in the right part of Fig. 48. On the other hand the echo readings due to catheter ringing as well as other noises are not influenced by the blood pulsation. Thus, the echo signal distance can be distinguished from the background by the fact that it moves backward and forward in correspondence to the blood pulsation. Thus, separating the signals that show the periodicity of the blood pressure movement from other noises may help to identify primary tissue echoes even though their signal to noise ratio is low. This allows accurate detection of the distance to the tissue wall.

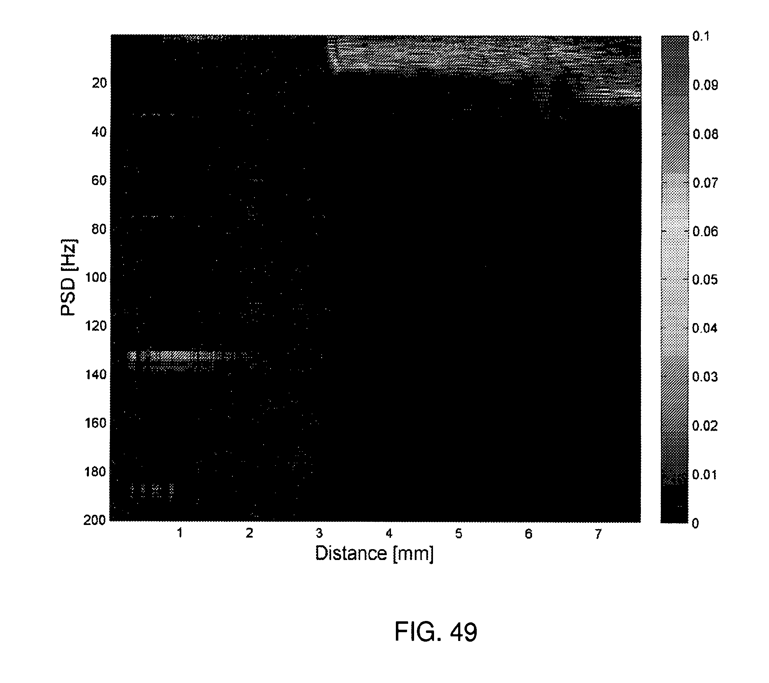

To do so, consecutive echo signals are first collected and the power spectrum is calculated for every point in the echo along consecutive trials. This is illustrated in figure 49 which shows the power spectrum of each point of the echo. Each point corresponds to a different distance. The power spectrum intensity is color coded from blue to red. Next, the ratio between the frequency components of breathing and blood pulsation, which were estimated as described earlier, and the frequency components of noise and ringing are calculated for each point of the echo signal. The response ratio is defined as the reflection profile and is used to separate tissue reflections from other noises.

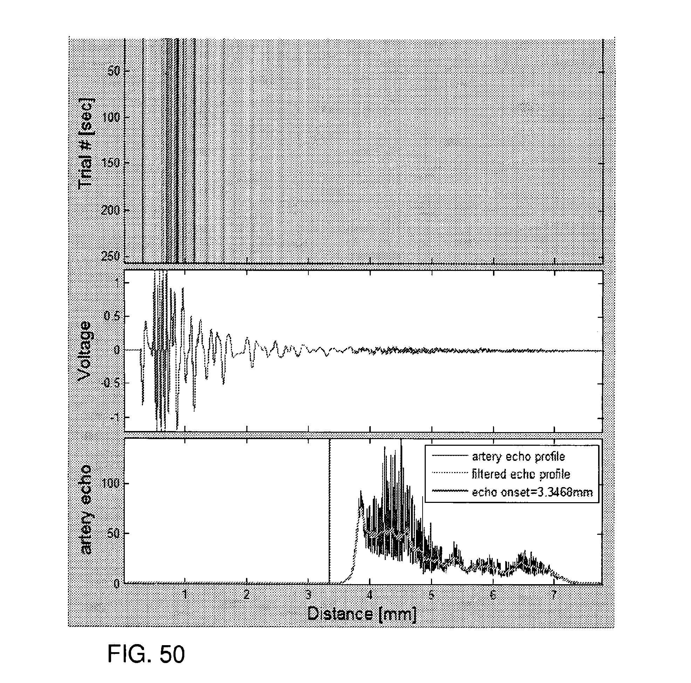

The results of such a process are illustrated in Fig. 50. The upper part of Fig. 50 shows 256 consecutive echo trials as a function of distance. Echo intensity is color coded from blue to red. The middle part shows five superimposed individual trials. Clearly, the variability profile between different signals during the ringing is different from the variability during reflections. These changes in variability are reflected in the power spectrum over consecutive echoes. The lower part of Fig. 50 is a plot of the reflection profile (in blue). A smoothed reflection profile (green) is obtained using a median filter. A rise in this profile indicates reflections from the tissue due to increase in frequencies associated with blood pressure and breathing. Finally, the distance from the artery can be identified using a threshold crossing of the smoothed reflection intensity function, the green line in Fig 50. The threshold is automatically detected by analyzing the spectrum of points with no tissue reflections, which points are detected using their low kurtosis values.

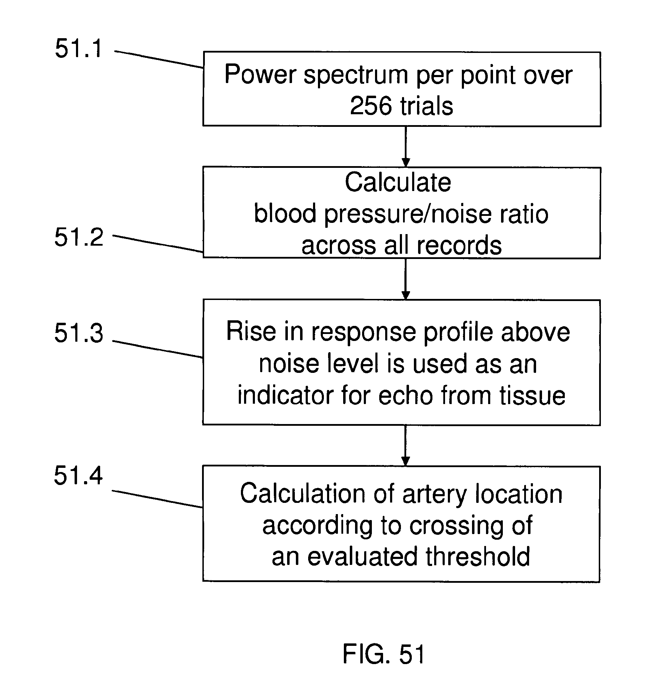

Referring now to Fig. 51, a summary of the procedure to achieve the above is as follows: Firstly, in 51.1, the power spectrum analysis over the same sample in consecutive echos is performed. In the given example (Fig 50), the echo signal is recorded 256 times, to form 256 records at a sampling frequency of 400 Hz. Within each record, 10000 points are sampled at 800 MHz.

A power spectrum is calculated for each point over the 256 consecutive trials, giving 10000 power spectrum graphs each being 256 samples in length. Following the calculation of the power spectra, the reflection intensity of every point is calculated by using the freq. ratio between the blood pressure frequencies and noise frequencies at each point (S51.2). Tissue reflections are identified by their high reflection intensity (S51.3). The tissue wall location is identified using an automatically detected threshold.

Although in the above, the Fourier component approach is presented as an alternative to coherent summation and convolution, it may also be used together with either or both of coherent summation and convolution to produce improved results.

Rationale

Reliable measurement may require transceiver - tissue boundary distances which are greater than 1 mm and up to 5 mm.

Signal acquisition is performed at 800 MHz/14 bit, and with a suitable oscilloscope such as that produced by Agilent™, the sampling rate may be increased to 2 GHz, but at a modest 8 bit resolution. With transceiver characteristic frequency about 10 and 20 MHz, time resolution is 80 - 40 samples per period.

Current implementations use PRF 400 Hz, which for 1000 repetitions constitutes a measurement duration of 2.5 s. This is enough to observe at least one entire period of heart pulse and therefore a full cycle of an artery dilating and shrinking. This linkage may facilitate distance measurement. In the experiments above there was processing of only 256 repetitions, using about 9000 samples each. At a PRF of 80 kHz, the entire measurement may take 3.2 ms. This time is too short for significant changes in mutual orientation of transceiver and tissue boundary, or distance between the transceiver and tissue boundary, and thus is expected to improve accuracy. In addition, a long acquisition process provides mutual incoherence of reflected signals which weakens the reflected signal level in the reference.

Currently the transceivers are narrow band. Their advantage is that the main component of their signal is at the Eigen frequency at different excitation shapes. Reflected signal appearance is seen clearly using the Hilbert transform and direct evaluation of local slopes of the phase - time curve. Another possibility is to create a Hilbert transform based instantaneous frequency. A special excitation shape may shorten transient reaction and therefore decrease the low measurement limit.

Due to averaging, a more stable excitation signal is summed almost coherently, while the reflected signal due to random phase variations is weakened with respect to the excitation signal. Thus simple averaging permits the creation of a reference which can be subtracted from a particular signal to almost eliminate the effects of the excitation signal. For better suppression of a (possible) narrow residual spike a low pass filter (LPF) may be applied. As a further point, due to malfunction or the like of an acquisition device, a portion of the signal that is intended for processing may sometimes contain high frequency oscillations between saturation levels. These oscillations are not the same for all particular signals and thus simple averaging is not efficient. In an embodiment, these spikes are identified, cut off, and interpolated with further optional filtration.

Although an Eigen frequency is supplied for the transceiver, it is reasonable to make a data-based estimation. Due to variable amplitude, an instantaneous frequency may change within certain limits even for a pure single-frequency signal. Thus, the frequency may be found from local slopes, which fall within predefined tolerances of the transceiver (say, $\pm 15\%$) and provide the smallest linear fitting errors. Parts of the data subjected to coherent summation are those which correspond to points which have a frequency that is close to the nominal transceiver frequency. Optionally, all entries of the instantaneous frequency matrix can be subjected to a logical operation which returns true if the frequency falls within the predetermined tolerances and false otherwise. A summation over the rows indicates how suitable a current point may be for coherent summation, and then a predetermined number of the points with largest sums are taken for coherent summation. A resonant window may then amplify true frequency components.

Phase correction of particular signals, that is bringing all signals of interest to the same phase, allows improvement in the SNR. Coherent summation is carried out twice: first, within groups of signals, for example eleven signals may be placed in a group; and then secondly over the groups themselves. Thus in an example of 23 groups, each with eleven signals, the summation would involve 11x23=253 signals. The obtained signal is convolved with a resonant window. Again, as discussed hereinabove, for better efficiency, groups may be arranged such that, within each group mutual closeness is better.

The above processing algorithm may provide a rapidly growing envelope signal. Since the slope is large, an error due to uncertainty of the threshold (signal appearance above noise) decreases.

The results of processing may show some cases of multiple reflections, which do not look as though they are reflections from the same object. It is likely that there are different objects with different reflecting properties. However the algorithm of the present embodiments provides a relative height and a length with each peak. The present embodiments may additionally help to classify/recognize detected objects.

It is expected that during the life of a patent maturing from this application many relevant ultrasound devices and transceivers will be developed and the scope of the corresponding terms used herein is intended to include all such new technologies *a priori*.

The terms "comprises", "comprising", "includes", "including", "having" and their conjugates mean "including but not limited to".

The term "consisting of" means "including and limited to".

As used herein, the singular form "a", "an" and "the" include plural references unless the context clearly dictates otherwise.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination or as suitable in any other described embodiment of the invention. Certain features described in the context of various embodiments are not to be considered essential features of those embodiments, unless the embodiment is inoperative without those elements.

## Claims

1. An ultrasonic transceiver apparatus for intracorporeal use, the apparatus comprising:

   an undamped ultrasonic transceiver adapted to be placed in a confined intracorporeal space, the transceiver having a characteristic frequency and being adapted to receive excitation at said characteristic frequency to produce an ultrasonic ablation beam for ablating surrounding tissues and being further adapted to receive excitation by primary echo signals returning from surrounding tissues;
   **characterised by** a signal processor connected to said transceiver configured to isolate said primary echo signals from ringing, secondary echoes and extraneous noise also received from said transceiver, said signal processor using correlation with a body-characteristic frequency as an isolation criterion.

2. The apparatus of claim 1, wherein said body characteristic frequency is one member of the group consisting of pulse and breathing rate.

3. The apparatus of claim 1, wherein said signal processor is configured to obtain a power spectrum of a signal extracted from said transceiver and to identify said primary echoes from peaks in said power spectrum at said body-characteristic frequency.

4. The apparatus of claim 1, further comprising a coherent summation unit.

5. The apparatus of claim 1, further comprising a convolution unit.

6. The ultrasonic transceiver apparatus of claim 1 comprising:

   circuitry which identifies locations of boundary walls of said tissue; and monitors changes in locations of said boundary walls as indicators of an effect of said energy on said tissue; and
   controls the applying of energy to tissue according to said effect.

7. The ultrasonic apparatus of claim 1, comprising circuitry for exciting said transceiver at an instantaneous excitation frequency to produce an ultrasonic ablation beam for ablating surrounding tissues using ablation pulses; and at intervals between said ablation pulses providing monitoring excitation to elicit primary echo signals returning from surrounding tissues.

8. The ultrasonic transceiver apparatus of claim 1, wherein said transceiver has an instantaneous excitation frequency and for receiving excitation at said excitation frequency to produce said ultrasonic ablation beam for ablating surrounding tissues; and wherein said signal processor uses presence or absence of said instantaneous excitation frequency as an isolation criterion.

9. The ultrasonic transceiver apparatus of claim 8, wherein said signal processor is configured with an instantaneous frequency estimator to obtain an envelope of received signal minus excitation signal from said undamped ultrasonic transceiver and to use a global phase and local slopes thereof as an estimate of said instantaneous frequency, and further comprising an isolator unit for isolating signal segments whose instantaneous frequency approaches said characteristic frequency as segments containing primary echoes.

10. The ultrasonic transceiver apparatus of claim 8 further comprising a window unit for windowing said received signal using a windowing length chosen to provide windows with an expectation of a single primary echo.

11. The ultrasonic receiving apparatus of claim 10, wherein said signal processor is further configured to find a point of appearance of a primary echo in a received signal by successively dividing said curve and fitting to a linear functions and calculating a point at which a corresponding error function is minimized.

12. The ultrasonic receiving apparatus of claim 11, further configured with a location unit to determine a distance to a first feature wall from said point of appearance and configured to use a second point of appearance of a further primary echo to determine a distance to a second feature wall, the signal processor further comprising a monitoring unit for monitoring a distance between said first feature wall and said second feature wall as an indicator of ablation progress.

13. The ultrasonic apparatus of claim 8, wherein said signal processor comprising a convolution unit for convolving an excitation signal with the received signal to carry out said isolation of the primary echo.

14. The ultrasonic apparatus of claim 8, further comprising a reference subtracting unit configured to subtract a reference from the transceiver signal by averaging several signal samples.

15. The apparatus of any of claims 1-14, wherein said ultrasonic ablation beam is not focused.

**Patentansprüche**

1. Ultraschall-Transceiver zur innerkörperlichen Verwendung, umfassend:

   einen ungedämpften Ultraschall-Transceiver, geeignet um in einen begrenzten innerkörperlichen Raum platziert zu werden, wobei der Transceiver eine charakteristische Frequenz aufweist und geeignet ist, Erregung bei der charakteristischen Frequenz zu empfangen, um einen Ablösestrahl zum Ablösen von umgebenden Gewebe zu erzeugen, und ferner geeignet ist, Erregung durch primäre Echosignale, zurückkommend von dem umgebenden Gewebe, zu empfangen;
   **gekennzeichnet durch** einen Signalprozessor, verbunden mit dem Transceiver, konfiguriert zum Isolieren der primären Echosignale von Lauten, sekundären Echos und belanglosen Geräuschen, ebenfalls von dem Transceiver empfangen, wobei der Signalprozessor eine Korrelation mit einer körpercharakteristischen Frequenz als Isolationskriterium verwendet.

2. Vorrichtung nach Anspruch 1, wobei die körpercharakteristische Frequenz ein Mitglied der Gruppe, bestehend aus Impuls und Atmungsrate, ist.

3. Vorrichtung nach Anspruch 1, wobei der Signalprozessor konfiguriert ist, ein Leistungsspektrum von einem Signal, extrahiert von dem Transceiver zu erhalten, und die primären Echos von Spitzen in dem Leistungsspektrum bei der körpercharakteristischen Frequenz zu identifizieren.

4. Vorrichtung nach Anspruch 1, ferner umfassend eine kohärente Summierungseinheit.

5. Vorrichtung nach Anspruch 1, ferner umfassend eine Windungseinheit.

6. Ultraschall-Transceiver-Vorrichtung nach Anspruch 1, umfassend:

   Schaltkreise zum Identifizieren der Lage von Begrenzungswänden des Gewebes; und zum Überwachen von Lageveränderungen der Begrenzungswände als Indikatoren für eine Auswirkung der Energie auf das Gewebe; und
   zum Steuern der Anwendung von Energie auf das Gewebe entsprechend der Auswirkung.

7. Ultraschallvorrichtung nach Anspruch 1, umfassend Schaltkreise zum Erregen des Transceivers bei einer Momentanerregungsfrequenz, um einen Ultraschall-Ablösungsstrahl zum Ablösen von umgebendem Gewebe unter Verwendung von Ablösungsimpulsen zu erzeugen; und
   in Intervallen zwischen den Ablösungsimpulsen eine Überwachung der Erregung bereitzustellen, um primäre Echosignale, zurückkommend von dem umgebenden Gewebe, auszulösen.

8. Ultraschall-Transceiver-Vorrichtung nach Anspruch 1, wobei der Transceiver eine Momentanerregungsfrequenz hat und zum Empfangen von Erregung bei der Erregungsfrequenz, um den Ultraschall-Ablösungsstrahl zum Ablösen des umgebenden Gewebes zu erzeugen; und wobei der Signalprozessor die Anwesenheit oder Abwesenheit der Momentanerregungsfrequenz als ein Isolationskriterium verwendet.

9. Ultraschall-Transceiver-Vorrichtung nach Anspruch 8, wobei der Signalprozessor mit einem Momentanfrequenzkalkulator konfiguriert ist, um eine Amplitude von empfangenem Signal minus Erregungssignal von dem ungedämpften Ultraschall-Transceiver zu erhalten und eine globale Phase und ein lokales Gefälle davon als eine Schätzung der Momentanfrequenz zu erhalten, und ferner umfassend eine Isolatoreinheit zum Isolieren von Signalsegmenten, deren Momentanfrequenz sich der charakteristischen Frequenz als Segmente enthaltende primäre Echos annähert.

10. Ultraschall-Transceiver-Vorrichtung nach Anspruch 8, ferner umfassend eine Fernstereinheit zur Fensterung des empfangenen Signals unter Verwendung einer Fensterungslänge, gewählt um Fenster mit einer Erwartung eines einzelnen Primärechos bereitzustellen.

11. Ultraschall-Empfangsvorrichtung nach Anspruch 10, wobei der Signalprozessor ferner konfiguriert ist, einen Erscheinungspunkt eines Primärechos in einem empfangenen Signal zu finden durch aufeinanderfolgendes Dividieren der Kurve, Anpassen an eine lineare Funktion und Berechnen eines Punktes, an dem eine entsprechende Fehlerfunktion minimiert wird.

12. Ultraschall-Empfangsvorrichtung nach Anspruch 11, ferner konfiguriert mit einer Lokalisierungseinheit zum Bestimmen eines Abstands zu einer ersten Merkmalswand von dem Erscheinungspunkt und konfiguriert zur Verwendung eines zweiten Erscheinungspunkts eines weiteren Primärechos zum Bestimmen eines Abstands zu einer weiteren Merkmalswand, wobei der Signalprozessor eine Überwachungseinheit zum Überwachen eines Abstands zwischen der ersten Merkmalswand und der zweiten Merkmalswand als ein Indikator des Ablösungsfortschritts umfasst.

13. Ultraschallvorrichtung nach Anspruch 8, wobei der Signalprozessor eine Windungseinheit zum Falten eines Erregungssignals mit dem empfangenen Signal umfasst, um die Isolierung des Primärechos zu bewerkstelligen.

14. Ultraschallvorrichtung nach Anspruch 8, ferner umfassend eine Referenz-Subtraktionseinheit, konfiguriert zum Subtrahieren einer Referenz von dem Transceiver-Signa durch Durchschnittsbestimmung mehrerer Signalproben.

15. Vorrichtung nach einem der Ansprüche 1-14, wobei der Ultraschall-Ablösestrahl nicht fokussiert ist.


## Revendications

1. Appareil émetteur récepteur d'ultrasons destiné à une utilisation intracorporelle, l'appareil comprenant :

   un émetteur récepteur d'ultrasons non amorti destiné à être placé dans un espace intracorporel confiné, l'émetteur récepteur présentant une fréquence propre, et étant adapté afin de recevoir une excitation à ladite fréquence propre de façon à produire un faisceau d'ultrasons d'ablation destiné à produire une ablation de tissus environnants, et étant en outre adapté afin de recevoir une excitation provoquée par les signaux d'écho primaires qui reviennent en provenance des tissus environnants ;
   **caractérisé par** un processeur de signaux connecté audit émetteur récepteur, configuré de façon à isoler lesdits signaux d'écho primaires des oscillations transitoires, des échos secondaires et du bruit parasite également reçus en provenance dudit émetteur récepteur, ledit processeur de signaux utilisant une corrélation avec une fréquence propre du corps en tant que critère d'isolement.

2. Appareil selon la revendication 1, dans lequel ladite fréquence propre du corps est un élément du groupe constitué par la fréquence du pouls et la fréquence respiratoire.

3. Appareil selon la revendication 1, dans lequel ledit processeur de signaux est configuré de façon à obtenir un spectre de puissance d'un signal extrait en provenance dudit émetteur récepteur, et à identifier lesdits échos primaires à partir des crêtes présentes dans ledit spectre de puissance à ladite fréquence propre du corps.

4. Appareil selon la revendication 1, comprenant en outre une unité de sommation cohérente.

5. Appareil selon la revendication 1, comprenant en outre une unité de convolution.

**6.** Appareil émetteur récepteur d'ultrasons selon la revendication 1, comprenant :

des circuits qui identifient des emplacements des parois limites dudit tissu ; et
qui surveillent les modifications des emplacements desdites parois limites en tant qu'indicateurs d'un effet de ladite énergie sur ledit tissu ; et
qui commandent l'application d'une énergie sur le tissu pour obtenir ledit effet.

**7.** Appareil à ultrasons selon la revendication 1, comprenant des circuits destinés à exciter ledit émetteur récepteur à une fréquence d'excitation instantanée de façon à produire un faisceau d'ablation d'ultrasons destiné à produire une ablation de tissus environnants en utilisant des impulsions d'ablation ; et
à des intervalles situés entre lesdites impulsions d'ablation, à fournir une excitation de surveillance de façon à obtenir des signaux d'écho primaires qui reviennent en provenance des tissus environnants.

**8.** Appareil émetteur récepteur d'ultrasons selon la revendication 1, dans lequel ledit émetteur récepteur présente une fréquence d'excitation instantanée et est destiné à recevoir une excitation à ladite fréquence d'excitation de façon à produire ledit faisceau d'ablation d'ultrasons destiné à produire une ablation de tissus environnants ; et dans lequel ledit processeur de signaux utilise la présence ou l'absence de ladite fréquence d'excitation instantanée en tant que critère d'isolement.

**9.** Appareil émetteur récepteur d'ultrasons selon la revendication 8, dans lequel ledit processeur de signaux est configuré avec un dispositif d'estimation de fréquence instantanée de façon à obtenir une enveloppe du signal reçu moins le signal d'excitation en provenance dudit émetteur récepteur d'ultrasons non amorti, et à utiliser une phase globale et des pentes locales s'y rapportant en tant qu'estimation de ladite fréquence instantanée, et comprenant en outre une unité d'isolement destinée à isoler lesdits segments de signal dont la fréquence instantanée approche ladite fréquence propre en tant que segments qui contiennent des échos primaires.

**10.** Appareil émetteur récepteur d'ultrasons selon la revendication 8, comprenant en outre une unité fenêtre destinée à placer dans une fenêtre ledit signal reçu en utilisant une longueur de fenêtre sélectionnée de façon à fournir des fenêtres dans lesquelles on attend un seul écho primaire.

**11.** Appareil émetteur récepteur d'ultrasons selon la revendication 10, dans lequel ledit processeur de signaux est configuré en outre de façon à trouver un point d'apparition d'un écho primaire dans un signal reçu en divisant de manière successive ladite courbe, et à la faire correspondre à des fonctions linéaires et à calculer un point où une fonction d'erreur correspondante est réduite au minimum.

**12.** Appareil émetteur récepteur d'ultrasons selon la revendication 11, configuré en outre avec une unité de localisation destinée à déterminer une distance jusqu'à une première paroi caractéristique à partir dudit point d'apparition, et configuré de façon à utiliser un deuxième point d'apparition d'un autre écho primaire de façon à déterminer une distance jusqu'à une deuxième paroi caractéristique, le processeur de signaux comprenant en outre une unité de surveillance destinée à surveiller une distance entre ladite première paroi caractéristique et ladite deuxième paroi caractéristique en tant qu'indicateur de la progression de l'ablation.

**13.** Appareil ultrasons selon la revendication 8, dans lequel ledit processeur de signaux comprend une unité de convolution destinée à exécuter une convolution entre un signal d'excitation et le signal reçu de façon à exécuter ledit isolement de l'écho primaire.

**14.** Appareil ultrasons selon la revendication 8, comprenant en outre une unité de soustraction de référence configurée de façon à soustraire une référence du signal de l'émetteur récepteur en moyennant plusieurs échantillons de signal.

**15.** Appareil selon l'une quelconque des revendications 1 à 14, dans lequel ledit faisceau d'ablation d'ultrasons n'est pas focalisé.

FIG. 1

Raw signal

Distance [mm]

Signal after reference subtarction

FIG. 2

FIG. 3

Raw signal after noise subtraction

Distance [mm]

Double coherent summation and convolution. Distance=3.32 mm

FIG. 4

EP 2 629 848 B1

# 0.69 mm

## FIG. 5A

Raw signal. Distance 0.69 mm

Phase of analytical signal

## FIG. 5B

EP 2 629 848 B1

# Two slopes. Estimated distance 0.88 mm vs. Actual 0.69 mm

Slopes: 1st 11.04 MHz, 2nd 10.93 MHz. Estimated distance 0.88 mm

Legend:
- ----- original
- ............ 1st fitting
- ——— 2nd fitting

Y-axis: Phase [rad]
X-axis: Distance [mm]

FIG. 6

Total fitting error

FIG. 7

# Local slopes

Local slopes of phase - distance curve

Legend:
- ⊘ slopes w/o smoothing
- — slopes with smoothing

X-axis: Distance [mm]

FIG. 8

# 1.19 mm

Raw signal. Distance 1.19 mm

FIG. 9A

Distance [mm]

Phase of analytical signal

FIG. 9B

# Estimated1.25 mm vs. Actual1.19 mm

Slopes: 1st 9.549 MHz, 2nd 9.277 MHz. Eestimated distance 1.25 mm

Legend:
- original
- 1st fitting
- 2nd fitting

Y-axis: Phase [rad]
X-axis: Distance [mm]

FIG. 10

Total fitting error

Total fitting error

Distance [mm]

FIG. 11

# Local slopes

FIG. 12

EP 2 629 848 B1

# 1.69 mm

EP 2 629 848 B1

Raw signal. Distance1.69 mm

FIG. 13A

Distance [mm]

Phase of analytical signal

Phase [rad]

FIG. 13B

# Estimated 1.61 mm vs. Actual 1.69 mm

Slopes: 1st 8.881 MHz, 2nd 9.672 MHz. Estimated distance 1.61 mm

FIG. 14

EP 2 629 848 B1

Total fitting error

FIG. 15

# Local slopes

FIG. 16

EP 2 629 848 B1

# 2.19 mm

Raw signal. Distance2.19 mm

FIG. 17A

Phase of analytical signal

FIG. 17B

# Estimated distance 1.96 mm vs. Actual 2.19 mm

Slopes: 1st 8.647 MHz, 2nd 10.54 MHz. Estimated distance 1.96 mm

Legend:
- original
- 1st fitting
- 2nd fitting

Y-axis: Phase [rad]
X-axis: Distance [mm]

FIG. 18

EP 2 629 848 B1

# Total fitting error

FIG. 19

Local slopes

Local slopes of phase - distance curve

* slopes w/o smoothing
— slopes with smoothing

Distance [mm]

FIG. 20

# 2.69 mm

EP 2 629 848 B1

FIG. 21A

Raw signal. Distance 2.69 mm

Distance [mm]

FIG. 21B

Phase of analytical signal

# Estimated distance 2.77 mm vs. Actual 2.69 mm

EP 2 629 848 B1

Slopes: 1st 8.622 MHz, 2nd 10.86 MHz. Estimated distance 2.77 mm

Legend:
- original
- 1st fitting
- 2nd fitting

Y-axis: Phase [rad]
X-axis: Distance [mm]

FIG. 22

# Total fitting error

EP 2 629 848 B1

FIG. 23

# Local slopes

FIG. 24

EP 2 629 848 B1

# 3.19 mm

Raw signal. Distance 3.19 mm

FIG. 25A

Phase of analytical signal

FIG. 25B

EP 2 629 848 B1

# Estimated distance 2.85 mm vs. Actual 3.19 mm

Slopes: 1st 8.686 MHz, 2nd 10.52 MHz. Estimated distance 2.85 mm

FIG. 26

EP 2 629 848 B1

Total fitting error

Total fitting error

Distance [mm]

FIG. 27

# Local slopes

FIG. 28

EP 2 629 848 B1

# 3.69 mm

EP 2 629 848 B1

**FIG. 29A**

Raw signal. Distance3.69 mm

Distance [mm]

**FIG. 29B**

Phase of analytical signal

# Two slopes. Estimated distance 3.66 mm vs. 3.69 mm

Slopes: 1st 8.47 MHz, 2nd 10.94 MHz. Estimated distance 3.66 mm

FIG. 30

EP 2 629 848 B1

# Total fitting error

FIG. 31

# Local slopes

Local slopes of phase - distance curve

FIG. 32

EP 2 629 848 B1

# Distance 4.19 mm

EP 2 629 848 B1

Raw signal. Distance 4.19 mm

FIG. 33A

Distance [mm]

Phase of analytical signal

FIG. 33B

# Estimated distance 3.83 mm vs. actual 4.19 mm

EP 2 629 848 B1

FIG. 34

# Total fitting error

FIG. 35

# Local slopes

FIG. 36

# 4.69 mm

Raw signal. Distance 4.69 mm

FIG. 37A

Distance [mm]

Phase of analytical signal

FIG. 37B

EP 2 629 848 B1

# . Estimated distance 4.36 mm vs. Actual 4.69 mm w/o outliers: 4.61 mm

Slopes: 1st 8.152 MHz, 2nd 9.484 MHz. Estimated distance 4.36 mm± 0.36 mm

FIG. 38

Total fitting error

Total fitting error

Distance [mm]

FIG. 39

# Local slopes

Local slopes of phase - distance curve

FIG. 40

EP 2 629 848 B1

# 5.19 mm

Raw signal. Distance 5.19 mm

FIG. 41A

Distance [mm]

Phase of analytical signal

FIG. 41B

EP 2 629 848 B1

# Estimated distance 4.83 mm vs. Actual 5.19 mm

Slopes: 1st 7.902 MHz, 2nd 9.873 MHz. Estimated distance 4.83 mm

FIG. 42

EP 2 629 848 B1

FIG. 43

# Local slopes

FIG. 44

EP 2 629 848 B1

# Summary

Summary of estimated distances. RMS error 0.24 mm, R=0.9954

y=0.89x+0.18

Estimated distance [mm]

Known distance [mm]

FIG. 45

EP 2 629 848 B1

**FIG. 46**

Raw Data

S1 — Hilbert transform

Analytic Signal

S2 — Reference signal construction

S3 — Reference subtraction

S4 — Averaging

S5 — New phase evaluation

S6 — Construct particular coherent signal

S7 — Actual Transducer Frequency Evaluation

S8 — Convolution with resonant Window

S9 — Threshold/distance Evaluation

EP 2 629 848 B1

EP 2 629 848 B1

S7.1 — Average phases of coherent signals

S7.2 — Divide phase curve and find fitting error per piece

S7.3 — Average local slopes for smallest errors to obtain Actual transceiver frequency

S7.4 — Resonant window construction

FIG. 47

The blood cycle frequency is: 77.9221 bpm

High pressure

Low pressure

Pressure shifts [mm]

Distance [mm]

FIG. 48

FIG. 49

EP 2 629 848 B1

FIG. 50

68

FIG. 51